# FASCICULE DE BREVET EUROPEEN

(11) **EP 1 178 039 B1**
(45) Date de publication et mention de la délivrance du brevet: **11.04.2007**
(21) Numéro de dépôt: 01420173.5
(22) Date de dépôt: 01.08.2001
(51) Int. Cl.: C07C 327/48, C07C 275/36, C07D 277/36, C07D 271/10, C07D 271/06, C07D 213/61, C07D 235/14, C07D 285/08, A01N 47/30, A01N 43/82, A01N 43/52

(54) **Dérivés de phényl(thio)urées et phényl(thio)carbamates fongicides**
Derivate von Phenyl(thio)harnstoff und Phenyl(thio)carbamat Fungiziden
Derivatives of phenyl(thio)urea and phenyl(thio)carbamate fungicides

(30) Priorité: 04.08.2000 FR 0010305
(43) Date de publication de la demande: 06.02.2002
(73) Titulaire: Bayer CropScience S.A., 69009 Lyon (FR)
(72) Inventeur: Gerusz, Vincent, 69009 Lyon (FR); Mansfield, Darren James, 69004 Lyon (FR); Perez, José, 69009 Lyon (FR); Tickle, David, Essex CM5 OHW (GB); Vors, Jean-Pierre, 69009 Lyon (FR); Baldwin, Derek, Essex CB10 1XL (GB); Hough, Thomas, Cambridge CB1 6LW (GB); Mitchell, Dale Robert, Chesterford, Essex CB10 1PB (GB)

(56) Documents cités:
- DE-A- 3 102 590
- US-A- 4 005 223
- US-A- 4 328 248
- US-A- 4 540 578
- US-A- 4 914 098
- US-A- 5 066 667
- US-A- 6 083 970

## Description

La présente invention concerne de nouveaux dérivés de phényl(thio)urées et phényl(thio)carbamates fongicides, leur procédé de préparation et les compositions fongicides qui les contiennent.

La demande de brevet WO 95/22532 décrit des phényltriazolinones possédant une activité herbicide. En particulier, est présenté le composé de formule (A), pour lequel aucune caractéristique n'est donnée. 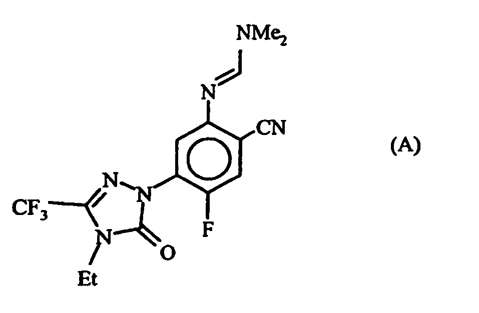 Le résumé et les revendications de composition et d'utilisation de cette demande se réfèrent uniquement à l'utilisation de tels composés pour des usages herbicides ; la description supporte en effet cette description uniquement avec des données d'activité herbicide. Il est simplement fait mention dans cette demande d'une possible activité fongicide pour certains composés. Cependant, aucune donnée d'activité fongicide n'est produite. Il n'y a aucune indication sur la nature des composés pouvant posséder une telle activité fongicide, et il n'y a aucune divulgation quant à l'activité fongicide du composé (A).

Par ailleurs, la publication DE-3102590 divulgue des composés dont la formule générale est la suivante : 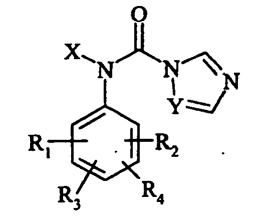 Les caractéristiques techniques essentielles pour des composés divulgués par ce document sont la présence d'une fonction ester et d'un groupement 1,3-pyrazole ou un 1,2,4-trizaole en présence d'un atome d'azote lié à un phényle substitué. De plus, les composés divulgués pour lesquels une activité biologique est rapportée possèdent tous cette fonction ester lié en position β par rapport à l'atome d'azote lié au au groupement phényle. Les deux exemples de composés 71 et 130 n'ont pas été synthétisés, ils n'ont donc pas pu être testés biologiquement.

La demanderesse a maintenant découvert que certains nouveaux carbamates, thiocarabamtes, urées et thio-urées, ainsi que leurs dérivés possèdent une activité fongicide. Ainsi, l'invention concerne les composés de formule générale (I) ainsi que leurs sels : 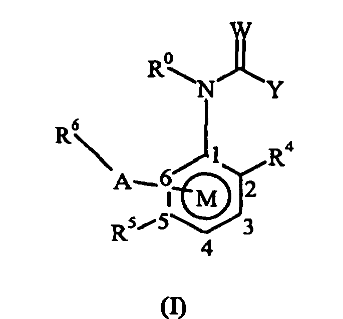 dans laquelle
- W représente O ou S ;
- Y représente un radical -NR¹R², -OR³ ou -SR³ ;
- R⁰ représente un radical alkyle ou l'atome d'hydrogène ;
- R¹ et R², qui peuvent être identiques ou différents, représentent un radical alkyle ou acyle chacun d'entre eux pouvant être substitué par un alkoxy ; ou l'atome d'hydrogène ; ou -NR^{a}R^{b} , -OR^{a} dans lesquels R^{a} et R^{b}, qui peuvent être identiques ou différents, représentent un radical alkyle, acyle ou carbocyclyle, chacun d'entre eux pouvant être substitué ;
- R³ représente un radical alkyle ou acyle chacun d'entre eux pouvant être substitué par un alkoxy ; ou l'atome d'hydrogène ;
- R¹ et R² ou R¹ et R⁰ ou R³ et R⁰ ou R^{a} et R^{b}, pris ensemble avec les atomes les connectant, peuvent former un cycle, éventuellement substitué, l'ensemble formant alors un groupement carbocyclyle ou hétérocyclyle ;
- R⁴ représente un radical alkyle pouvant être substitué par un atome d'halogène ;
- R⁵ représente un radical alkyle ou acyle chacun d'entre eux pouvant être substitué; ou l'atome d'hydrogène ;
- R⁶ représente un radical phényle ou un radical hétérocyclyle aromatique ;
- A représente -O- ou -S-.

Selon un aspect préféré, l'invention concerne les composés de formule générale (I) et les sels de ceux-ci dans lesquels :
- R⁰ représente un radical alkyle ou représente l'atome d'hydrogène ;
- R¹, R² et R³ sont tels que définis précédemment ;
- R⁴ représente un radical alkyle pouvant être substitué par un atome d'halogène ;
- A représente les radicaux divalents -O- ou -S- ; et
- R⁶ représente un radical phényle ou hétérocyclyle aromatique (de préférence thiazolyle, isothiazolyle, thiadiazolyle, pyridyle ou pyrimidinyle), éventuellement substitué par un ou plusieurs substituants, qui peuvent être identiques ou différents, et sont choisis parmi hydroxy, atome d'halogènes, cyano, acyle (de préférence -C(=O)R^{c}, -C(=S)R^{c} ou -S(O)ₚR^{c}, dans lesquels p représente 0, 1 ou 2 et R^{c} représente alkyle, haloalkyle, alkoxy, haloalkoxy, alkylthio, amino, monoalkylamino, dialkylamino ou phényle éventuellement substitué par alkyle, haloalkyle, alkoxy, haloalkoxy ou alkylthio ; ou phényloxy, phénylthio, carbocyclyle ou hétérocyclyle), amino, alkylamino, dialkylamino, alkyle, alkényle, alkynyle, haloalkyle, R^{a}O-alkyle, acyloxyalkyle, cyano-oxyalkyle, alkoxy, haloalkoxy, alkylthio, carbocyclyle (de préférence cyclohexyle ou cyclopentyle) éventuellement substitué par alkyle, haloalkyle, alkoxy, haloalkoxy ou alkylthio ; et benzyle éventuellement substitué par alkyle, haloalkyle, alkoxy, haloalkoxy ou alkylthio.

Selon un aspect particulièrement préféré de l'invention, celle-ci concerne les composés de formule générale (I), et leurs sels, tels que définis précédemment, et possédant les caractéristiques suivantes prises isolément ou en combinaison :
- R⁴ représente un radical alkyle en C₁-C₁₀ ;
- R⁵ représente un radical alkyle en C₁-C₁₀ ;
- A représente une liaison directe ou le radical divalent -O-, et occupe la position 4 sur le noyau benzénique M ;
- R⁶ représente un radical phényle éventuellement substitué par un ou plusieurs substituants pouvant être halogènes, alkyle, alkényle, alkynyle, haloalkyle, alkoxyalkyle, hydroxyalkyle, haloalkoxyalkyle, alkoxy, alkylthio, acyle ou cyano.

Des complexes des composés selon l'invention peuvent être formés de manière usuelle à partir d'un sel de formule NAn ou NAn₂, dans lequel N représente un cation métallique, par exemple cuivre, manganèse, cobalt, nickel, fer ou zinc et An représente un anion, par exemple chlorure, nitrate ou sulfate.

Les N-oxydes des composés de l'invention, lorsque ceux-ci comprennent un atome d'azote pouvant être oxydé, sont également compris dans le champ de la présente invention.

**[00010]** Dans les cas où les composés selon l'invention existent sous formes d'isomères géométriques E et Z, l'invention comprend les isomères individuels ainsi que les mélanges de ceux-ci en toutes proportions.

**[00011]** Dans les cas où les composés selon l'invention existent sous formes d'isomères tautomériques, l'invention comprend les isomères individuels tautomériques ainsi que les mélanges de ceux-ci en toutes proportions.

Dans les cas où les composés selon l'invention existent sous formes d'isomères optiques, l'invention comprend les isomères individuels ainsi que les mélanges de ceux-ci en toutes proportions, y compris le mélange 50:50, dit mélange racémique.

Tout groupement alkyle défini ci-dessus peut être linéaire ou ramifié et comprend généralement de 1 à 10 atomes de carbone, de préférence de 1 à 7 atomes de carbone, de préférence encore de 1 à 5 atomes de carbone.

Tous les groupements alkényle ou alkynle définis ci-dessus peuvent être linéaires ou ramifiés, comprennent de préférence de 2 à 7 atomes de carbone, et contiennent généralement jusqu'à 3 doubles ou triples liaisons qui peuvent être conjuguées, par exemple vinyle, allyle, butadiényle ou propargyle.

Les groupements carbocyclyle peuvent être saturés, insaturés ou aromatiques et contenir de 3 à 8 chaînons. Des groupements carbocyclyle saturés préférés comprennent cyclopropyle, cyclopentyle, et cyclohexyle. Des groupements carbocyclyle insaturés préférés comprennent jusqu'à 3 doubles liaisons. Un groupement carbocyclyl aromatique préféré et phényle. Le terme carbocyclic possède la même définition dans la présente invention. De plus, le terme carbocyclyle comprend tout type de groupements carbocyclyle fusionnés, tels que naphtyle, phénantryle, indanyle et indényle.

Les groupements hétérocyclyle peuvent être saturés, insaturés ou aromatiques et contenir de 3 à 7chaînons, parmi lesquels jusqu'à 4 d'entre eux peuvent être des hétéroatomes tels que azote, oxygène et soufre. Ainsi, par groupement hétérocyclyle, on entend par exemple furyle, thiényle, pyrrolyle, pyrrolinyle, pyrrolidinyle, imidazolyle, dioxolanyle, oxazolyle, thiazolyle, imidazolinyle, imidazolidinyle, pyrazolyle, pyrazolinyle, pyrazolidinyle, isoxazolyle, isothiazolyle, oxadiazolyle, triazolyle, thiadiazolyle, pyranyle, pyridyle, pipéridinyle, dioxanyle, morpholino, dithianyle, thiomorpholino, pyridazinyle, pirimidinyle, pyrazinyle, pipérazinyle, sulfolanyle, tétrazolyle, triazinyle, azépinyle, oxazépinyle, thiazépinyle, diazépinyle et thiazolinyle.

De plus, le terme hétérocyclyle comprend les groupements hétérocyclyle fusionnés, par exemple benzimidazolyle, benzoxazolyle, imidazopyridinyle, benzoxazinyle, benzothiazinyle, oxazolopyridinyle, benzofuranyle, quinolinyle, quinazolinyle, qionoxalinyle, dihydroquinazolinyle, benzothiazolyle, phtalimido, benzofuranyle, benzodiazépinyle, indolyle et isoindolyle. Le terme hétérocyclique possède les mêmes définitions.

L'adjectif "substitué" qualifiant les groupements alkyle, alkényle, alkynyle, carbocyclyle et hétérocyclyle signifie que ces groupements peuvent être substitués par un ou plusieurs substituants, identiques ou différents, choisis parmi : hydroxy, alkényle, alkynyle, mercapto, azido, nitro, halogènes, cyano, acyle, alkoxycarbonyle, aminocarbonyle éventuellement substitué, amino éventuellement substitué, ammonio éventuellement substitué, carbocyclyle éventuellement subtitué, hétérocyclyle éventuellement subtitué, cyanato, thiocyanato, -SF₅; -OR^{a}; -SR^{a}; -SOR^{a}; -SO₂R^{a} et -Si(R^{a})₃, avec R^{a} étant alkyle, alkényle, alkynyle, carbocyclyle or hétérocyclyle, chacun d'eux pouvant être éventuellement substitué. Pour les groupements carbocyclyle et hétérocyclyle la liste comprend de plus alkyle, alkényle et alkynyle, chacun d'eux pouvant être éventuellement substitué. De substituants préférés pour les radicaux alkyle, alkényle ou alkynyle sont : alkoxy, haloalkoxy ou alkylthio, chacun d'eux contenant de 1 à 5 atomes de carbone ; halogène; ou phényle éventuellement substitué. Des substituants préférés pour les groupements carbocyclyle et hétérocyclyle sont : alkyle, haloalkyle, alkoxy, haloalkoxy ou alkylthio, chacun d'eux contenant de 1 à 5 atomes de carbone; halogène; ou phényle éventuellement substitué.

Pour les radicaux alkyle et les atomes de carbone des groupements carbocyclyle et hétérocyclyle, la liste comprend les radicaux divalents tels que oxo ou imino, éventuellement substitué par amino éventuellement substitué, R^{a} or -OR^{a} (où R^{a} est tel que défini précédement). Des radicaux préférés sont oxo, imino, alkylimino, oximino, alkyloximino ou hydrazono.

Les groupements amino, lorsqu'ils sont substitués et le cas échéant, peuvent être substitués par un ou deux substituants identiques ou différents, choisis parmi : alkyle, alkényle, alkynyle éventuellement substitué, amino éventuellement substitué, -OR^{a} (où R^{a} est tel que défini précédemment), carbocyclyle, hétérocyclyle et acyle. Selon un autre aspect, deux substituants, avec l'atome d'azote auxquels ils sont liés, peuvent former un groupement hétérocyclyle, de préférence un groupement hétérocyclyle contenant de 5 à 7 chaînons, qui peut être substitué et qui peut contenir d'autres hétéroatomes, par exemple morpholino, thiomorpholino ou pipéridinyle.

Le terme acyle comprend les résidus d'acides sulfurés et phosphorés et également d'acides carboxyliques. Les dits résidus sont ainsi compris dans les formules générales :-C(=X^{a})R^{b}, -S(O)ₚR^{b} et -P(=X^{a})(OR^{a})(OR^{a}), où, de façon appropriée, X^{a} représente O ou S, R^{b} est tel que défini pour R^{a}, -OR^{a}, -SR^{a}, amino éventuellement substitué ou acyle; et p représente 1 ou 2. Des groupements préférés sont -C(=O)R^{c}, -C(=S)R^{c}, et -S(O)ₚR^{c} dans lesquels R^{c} représente alkyle, C₁-C₅ alkoxy, C₁-C₅ alkylthio, phényle, phényloxy, phénylthio, carbocyclyle, hétérocyclyle ou amino, chacun d'entre eux pouvant être éventuellement substitué.

Les composés selon l'invention sont utiles comme fongicides et contrôlent les pathogènes des familles Deuteromycètes, Oomycètes, Ascomycètes, Phycomycètes et Basidiomycètes. Une activité antifongique est mise en évidence sur les maladies des céréales telles que, par exemple :
- les SEPTORIOSES du blé (*Leptosphaeria nodorum & Septoria tritici*),
- les OÏDIUMS (*Erysiphe graminis f.sp hordei et Erysiphe graminis f.sp tritici),*
- les ROUILLES du blé (*Puccinia recondita*, *Puccinia striiformis*),
- le PIÉTIN-VERSE du blé (*Pseudocercosporella herpotrichoides*),
- l'HELMINTHOSPORIOSE de l'orge *(Drechslera teres)* et
- la RHYNCHOSPORIOSE de l'orge (*Rhynchosporiose secalis*).
De même :
- les MILDIOUS des solanées (*Phytophthora infestans*) et de la vigne *(Plasmopara viticola*),
- la POURRITURE GRISE (*Botrytis cinerea*),
- les maladies du riz (PYRICULARIOSE, PELLICULARIOSE, RHIZOCTONE)
sont contrôlés.

Ainsi la présente invention fournit également une méthode pour combattre les champignons phytopathogènes en un lieu qui en est infesté ou susceptible de l'être, qui comprend l'application en ce lieu d'un composé de formule (I) ou d'un de ses sels.

L'invention fournit également une composition pour l'agriculture comprenant un composé de formule (I) ou un des ses sels en mélange avec un diluant ou un support acceptable en agriculture.

La composition selon l'invention peut bien sûr comprendre plus d'un composé selon l'invention.

De plus, la composition peut comprendre une ou plusieurs matières actives additionnelles, par exemple des composés connus pour posséder des propriétés de régulateur de croissance des plantes, des propriétés herbicides, fongicides, insecticides, acaricides, antimicrobiennes ou antibactériennes. Le composé selon l'invention peut être utilisé ainsi, par exemple, de manière simultanée, séquentielle ou alternative avec la ou les autre(s) matière(s) active(s).

Le diluant ou le support dans la composition selon l'invention peut être un solide ou un liquide en association avec un agent tensioactif, par exemple un agent dispersant, un agent émulsionnant ou un agent mouillant. Des agents tensio-actifs convenables comprennent des composés anioniques tels que un carboxylate, par exemple un carboxylate métallique d'un acide gras à longue chaîne ; un *N*-acylsarcosinate ; des mono- ou di-esters d'acide phosphorique avec des éthylates d'alcools gras ou des éthylates d'alkyle phénol ou des sels de tels esters ; des sulfates d'alcools gras tels que du sulfate de dodécyle sodium, du sulfate d'octadécyle sodium ou du sulfate de cétyle sodium; des sulfates d'alcools gras éthoxylés ; des sulfates d'alkyle phénol éthoxylés ; des sulfonates de lignine; des sulfonates de pétrole ; des alkyle-aryle sulfonates tels que des alkyl-benzène sulfonates ou des alkyl inférieur naphtalène sulfonates, par exemple du butyl-naphtalène sulfonate ; des condensats de sels de naphtalène sulfone-formaldéhyde ; des condensats de sels de phénol sulfone-formaldéhyde; ou des sulfonates plus complexes tels que des sulfonates d'amide, par exemple le produit de condensation de l'acide oléique et de la *N*-méthyl taurine sulfoné ; des dialkyl-sulfosuccinates, par exemple le sulfonate de sodium du dioctyl-succinate ; des dérivés acides de composés alkylglycosides et alkylpolyglycosides et leurs sels métalliques, par exemple citrate ou tartrate d'alkylpolyglycoside; ou mono-, di- et tri-alkyl esters d'acide citrique et leurs sels métalliques.

Des agents non ioniques comprennent des produits de condensation d'esters d'acides gras, d'alcools gras, d'amides d'acides gras ou de phénols substitués par des alkyl- ou alkényl-gras avec de l'oxyde d'éthylène et/ou de propylène ; des esters gras d'éthers d'alcools polyhydriques, par exemple des sorbitan esters d'acides gras ; des produits de condensation de tels esters avec de l'oxyde d'éthylène, par exemple des polyoxyéthylène sorbitan esters d'acides gras ; des produits alkyl glycosides, des produits alkyl polyglycosides ; des copolymères blocs d'oxyde d'éthylène et d'oxyde de propylène ; des glycols acétyléniques tels que le 2,4,7,9-tétraméthyl-5-décyne-4,7-diol ; des glycols acétyléniques éthoxylés ; des copolymères greffés à maillon acrylique ; des surfactants siloxane alkoxylés ; ou des surfactants de type imidazoline, par exemple la 1-hydroxyéthyl-2-alkylimidazoline.

Des exemples d'agents tensioactifs cationiques comprennent, par exemple, une mono-, une di- ou une polyamine, sous forme d'acétate, de naphténate ou d'oléate; une amine contenant de l'oxygène, comme un oxyde d'amine, une polyoxyéthylène-alkylamine ou une polyoxypropylène-alkylamine; une amine liée à une amide préparée par condensation d'un acide carboxylique avec une di- ou une polyamine ; ou un sel d'ammonium quaternaire.

Les compositions selon l'invention peuvent prendre toute forme dans le domaine de la formulation de composés agrochimiques, par exemple, solution, aérosol, dispersion, émulsion aqueuse, micro émulsion, concentré dispersable, poudre à pulvériser, composition pour enrobage ou pelliculage de semences, composition pour fumigation ou pour enfumage, poudre dispersable, concentré émulsifiable, granulés ou bande imprégnée. De plus, elles peuvent être sous une forme convenant à l'application directe ou comme concentré ou composition primaire nécessitant dilution avec une quantité convenable d'eau ou d'un autre diluant avant application.

Un concentré dispersable comprend un composé selon l'invention, dissous dans un ou plusieurs solvants solubles en totalité ou en partie dans l'eau, et un ou plusieurs agents tensioactifs et/ou polymériques. L'addition de la formulation dans l'eau provoque une cristallisation de la matière active, le processus étant contrôlé par les tensioactifs et/ou les polymères, ce qui conduit à une dispersion fine.

Une poudre pulvérisable comprend un composé selon l'invention mélangé et broyé intimement avec un diluant solide pulvérulent, par exemple, le kaolin.

Un concentré émulsifiable comprend un composé selon l'invention dissous dans un solvant non miscible à l'eau et qui forme une émulsion ou une micro émulsion lors de l'addition dans de l'eau en présence d'un agent émulsifiant.

Un granulé solide comprend un composé selon l'invention associé à des diluants similaires à ceux pouvant être employés pour les poudres pulvérisables, le mélange étant ici granulé selon des méthodes connues. Selon une alternative, le granulé solide comprend la matière active absorbée ou enrobée sur un support granulé préformé, par exemple, la terre de Fuller, l'attapulgite, la silice ou le sable calcaire ("limestone grit" en langue anglaise).

Les poudres mouillables, granules ou granulés, comprennent habituellement la matière active en mélange avec des tensioactifs convenables et un diluent en poudre inerte tel que l'argile ou la terre diatomacée.

Un autre concentré convenable est une suspension concentrée coulable formée par broyage du composé avec de l'eau ou un autre liquide, des tensioactifs et un agent de suspension.

La concentration de la matière active dans la composition de la présente invention, telle qu'appliquée aux plantes, est de préférence comprise entre 0,0001 et 1,0 pour cent en poids, particulièrement entre 0,0001 et 0,01 pour cent en poids. Dans une composition primaire, la quantité de matière active peut varier largement et peut, par exemple, être comprise entre 5 et 95 pour cent en poids de composition.

Lorsqu'il est utilisé, un composé de l'invention est généralement appliqué aux semences, aux plantes ou leur habitat. Ainsi, le composé peut être appliqué directement sur le sol avant, au moment ou après l'ensemencement de sorte que la présence de la matière active dans le sol peut contrôler la croissance des champignons qui peuvent attaquer les semences. Lors d'un traitement direct du sol, la matière active peut être appliquée de quelque manière que ce soit qui lui permette d'être intimement mélangée au sol telle que par pulvérisation, par diffusion de granulés sous forme solide ou par application de la matière active au même moment que la semence en l'utilisant dans le même semoir que les graines. Une dose d'application convenable est comprise entre 5 et 1000 g par hectare, de préférence encore entre 10 et 500 g par hectare.

De manière alternative la matière active peut être appliquée directement à la plante, par exemple par pulvérisation ou poudrage soit au moment où le champignon a commencé à apparaître sur la plante soit avant l'apparition du champignon comme mesure de protection. Dans ces deux cas le mode d'application préféré est une pulvérisation sur les feuilles. Il est généralement important d'obtenir une lutte efficace contre les champignons aux stades précoces de croissance de la plante, puisque c'est le moment où la plante peut être le plus sévèrement endommagée. La pulvérisation ou le poudrage peut de manière commode contenir un herbicide de pré- ou post-émergence si cela est cependant nécessaire. Parfois, il est faisable de traiter les racines, les bulbes, les tubercules ou autres excroissances végétales d'une plante avant ou au cours de la plantation, par exemple, en trempant les racines dans une composition liquide ou solide convenable. Lorsque la matière active est appliquée , directement à la plante une dose d'application convenable est comprise entre 0,025 et 5 kg par hectare, de préférence entre 0,05 et 1 kg par hectare.

De plus, les composés selon l'invention peuvent être appliqués aux fruits, légumes ou graines récoltés pour prévenir l'infection pendant le stockage.

De plus, les composés selon l'invention peuvent être appliqués aux plantes ou parties de celles-ci qui ont été génétiquement modifiées.

De plus, les composés selon l'invention peuvent être utilisés pour traiter des infections fongiques dans le bois de construction et pour application à la santé publique. Les composés selon l'invention peuvent également être utilisés pour traiter des infections fongiques sur des animaux domestiques ou de ferme.

Des composés selon l'invention peuvent être préparés par de nombreuses voies, selon des procédés connus.

Ainsi, les composés de formule générale (I) peuvent par exemple être préparés à partir de composés de formule générale (II) selon le Schéma 1 suivant :

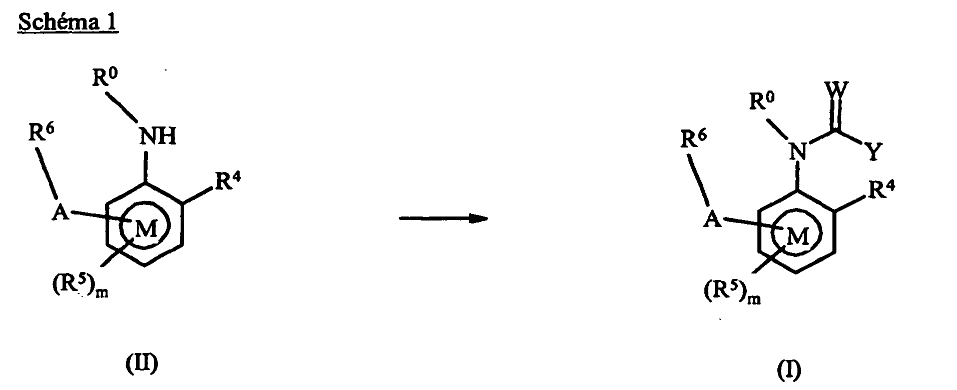

Les conditions de réaction suivantes peuvent être utilisées pour réaliser la conversion de composés de formule (II) en composés de formule (I):
- de manière générale, réaction avec Cl(CW)Y, c'est-à-dire un chlorure de carbamoyle lorsque W représente O et Y représente -NR¹R², un chlorure de thiocarbamoyle lorsque W représente S et Y représente -NR¹R², un chloroformiate lorsque W représente O et Y représente -OR³, un chlorothionoformiate lorsque W représente S et Y représente -OR³, un chlorothiolformiate lorsque W représente O et Y représente -SR³ ou un chlorodithioformiate lorsque W représente S et Y représente -SR³ ;
- dans le cas où R⁰ représente l'atome d'hydrogène et W représente O, réaction avec du phosgène, diphosgène ou triphosgène pour former l'isocyanate et ensuite traitement avec HY ;
- dans le cas où R⁰ représente l'atome d'hydrogène et W représente S, réaction avec du thiophosgène pour former l'isothiocyanate et ensuite traitement avec HY ;
- dans le cas où Y représente -NHR¹, réaction avec R¹N=(CW);
- dans le cas où W représente S, Y représente -SR³ et R⁰ est différent de l'atome d'hydrogène, réaction avec le disulfure de carbone et R³X, X représentant un atome d'halogènes ou tout autre groupement partant.

De plus, des composés de formule générale (I) peuvent être convertis en d'autres composés de formule générale (I) par dérivatisation des radicaux R⁰ ou R¹ ou R² ou R³ ; ou par acylation, cyanation ou alkylation lorsque W représente O et R⁰ ou R¹ ou R² ou R³ représentent l'atome d'hydrogène ;

Dans le cas où R⁰ est différent de l'atome d'hydrogène, des composés de formule générale (II) peuvent être préparés à partir de composés de formule générale (III) selon le Schéma 2 suivant :

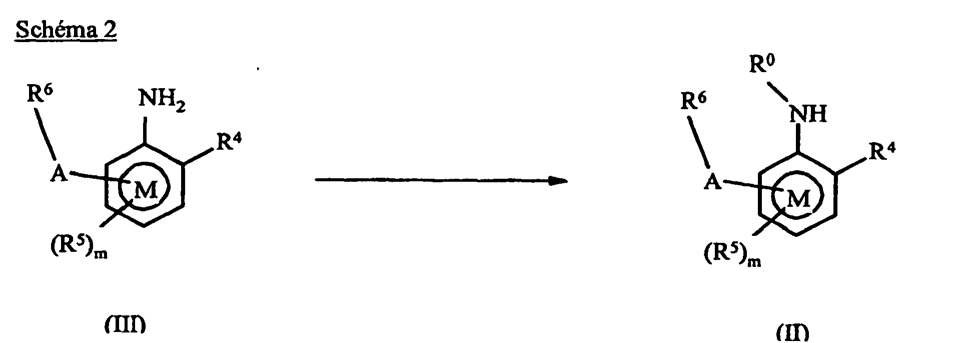

Les conditions de réaction suivantes peuvent être utilisées pour réaliser la conversion :
- dans le cas où R⁰ représente un radical alkyle, formylation ou acylation par un anhydride suivie d'une réduction par le diméthylsulfure de borane ;
- dans le cas où R⁰ représente un radical cyano, cyanation au chlorure ou bromure de cyanogène ;
- dans le cas où R⁰ représente un radical acyle, acylation par un halogénure d'acyle.

Les composés de formule générale (III) peuvent être préparés par réduction du radical nitro des composés de formule (IV) selon le Schéma 3. Les conditions de réaction préférées comprennent une réaction avec l'hydrate d'hydrazine dans de l'éthanol catalysée par du palladium ou du platine ;

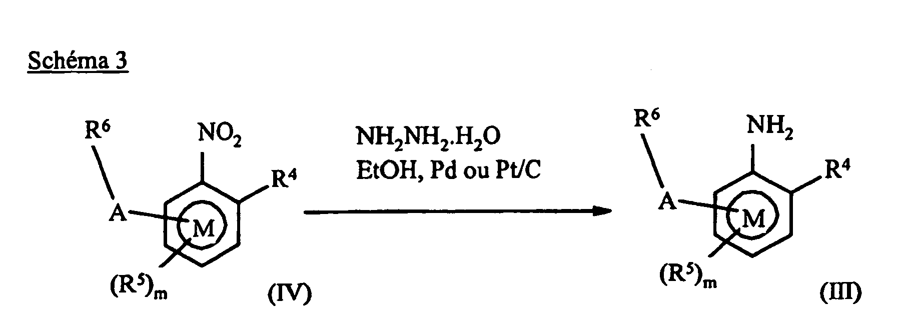

Les composés de formule (IIIa), c'est-à-dire les composés de formule générale (III) dans laquelle A représente une liaison directe, peuvent être préparés selon le Schéma 4, dans lequel X^{V} représente un groupe partant :

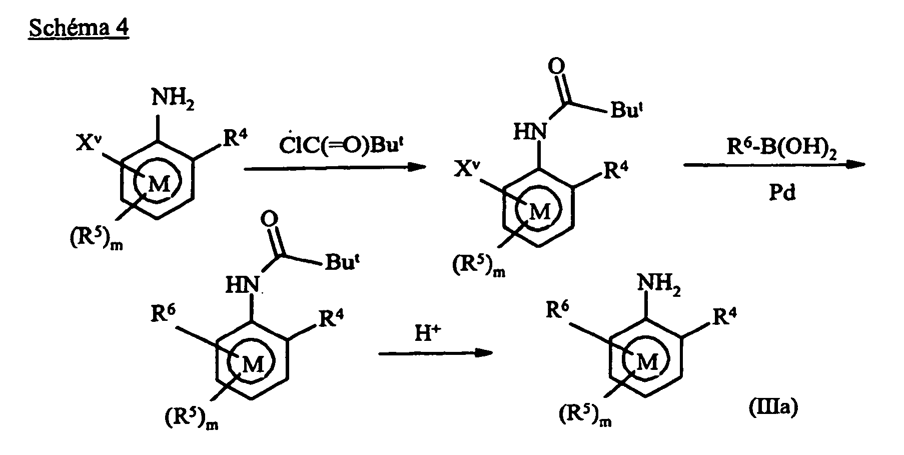

Les composés de formule (IIIb), c'est-à-dire les composés de formule générale (III) dans laquelle R⁴ représente un atome d'halogènes, peuvent être préparés selon le Schéma 5, dans lequel X^{T} représente un atome d'halogènes. Lorsque X^{T} représente le brome les conditions de réaction préférées comprennent l'agitation avec du brome dans un solvant convenable.

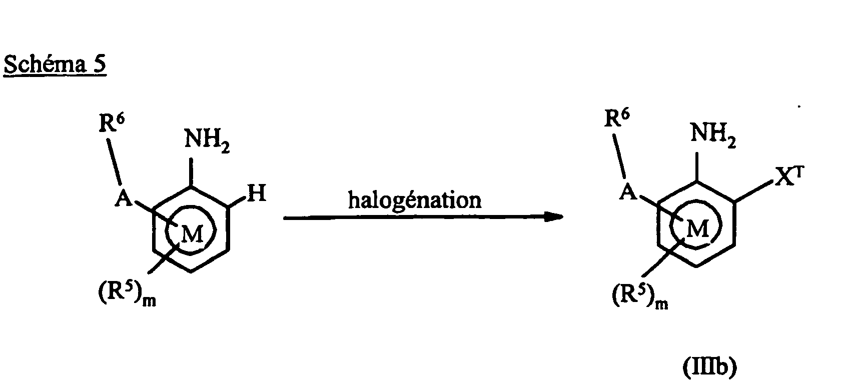

Les composés de formule (IIIc), c'est-à-dire les composés de formule générale (III) dans laquelle A représente NHC(=O)- ; les composés de formule (IIId), c'est-à-dire les composés de formule (III) dans laquelle A représente une liaison directe et R⁶ représente le radical phtalimido éventuellement substitué, dans lequel la ligne courbe connectant les positions 3 et 4 du radical phtalimido représente la chaîne carbocyclique éventuellement substituée ; et les composés de formule (IIIe), c'est-à-dire les composés de formule générale (III) dans laquelle A représente une liaison directe et R⁶ représente le radical pyrrolyle, éventuellement substitué en positions 2 et 5 par un ou plusieurs radicaux R qui peuvent être identiques ou différents ; peuvent être préparés à partir de composés de formule (V) selon la méthodologie présentée dans le Schéma 6. Pour certains composés de formule (V), la protection/déprotection du radical amino en position ortho du radical R⁴ peut être requise pour améliorer les rendements.

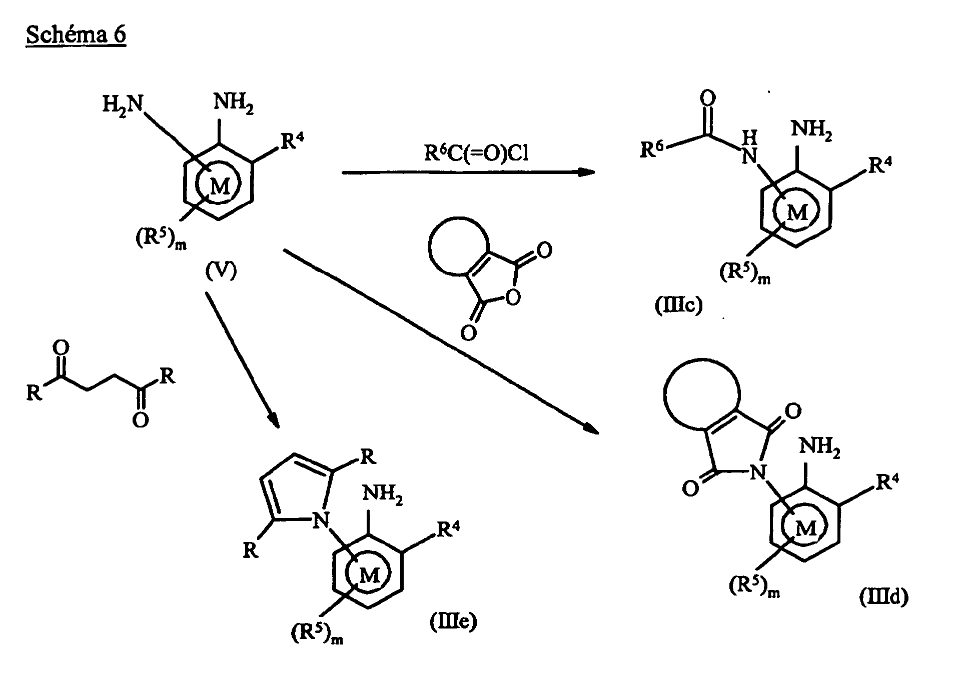

Les composés de formule (IVa), c'est-à-dire les composés de formule générale (IV) dans laquelle A représente un radical A^{Z}, peuvent être préparés en faisant réagir les composés de formule (VI) avec les composés de formule (VII) selon le Schéma 7. A^{Z} représente un radical qui, dans le composé (VI), forme un anion dans des conditions basiques. A^{Z} représente de manière alternative un atome d'azote primaire ou secondaire basique. X^{Z} représente un groupe partant, de préférence un atome d'halogènes. Lorsque A^{Z} représente l'atome d'oxygène, des conditions de réaction préférées comprennent le traitement de (VI) avec de l'hydrure de sodium suivi de l'addition de (VII). Lorsque A^{Z} représente l'atome de soufre, les conditions de réaction préférées comprennent la réaction de (VI) avec (VII) en présence d'une base amine tertiaire telle que l'éthyldiisopropylamine. Lorsque A^{Z} représente -CHR⁷-, les conditions de réaction préférées comprennent le traitement de (VI) avec du *tert-*butylate de potassium dans de la diméthylformamide à basse température. Lorsque A^{Z} représente un atome d'azote basique, aucune base n'est requise.

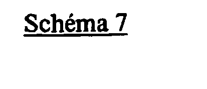

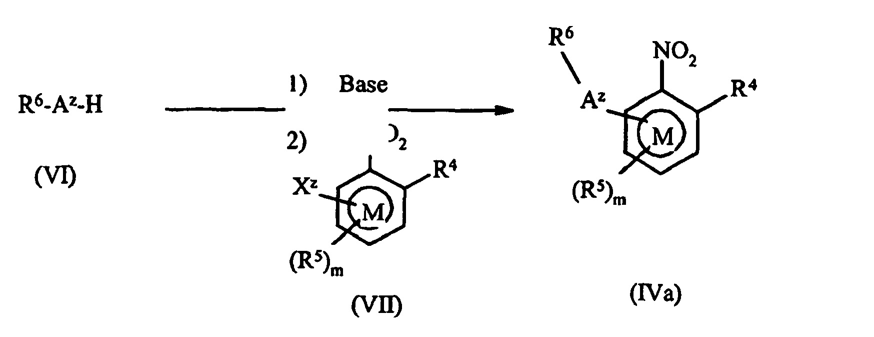

Les composés de formule (IVb), c'est-à-dire les composés de formule générale (IV) dans laquelle A représente un radical A^{W}, peuvent être préparés par réaction de composés de formule (VIII) avec les composés de formule (IX) selon le Schéma 8. A^{W} représente un radical qui, dans un composé (VIII), forme un anion dans des conditions basiques. X^{W} représente un groupe partant, de préférence un atome d'halogènes. Des conditions basiques préférées comprennent la réaction de (VIII) avec du carbonate de potassium carbonate ou de l'hydrure de sodium suivie de l'addition de (IX).

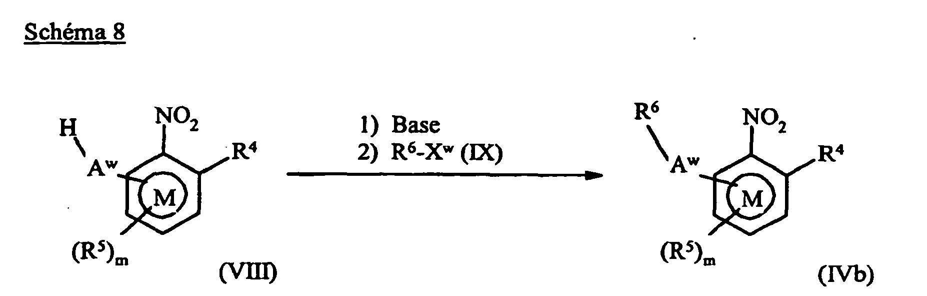

Les composés de formule (IVc), c'est-à-dire les composés de formule générale (IV) dans laquelle A représente O, peuvent être préparés par réaction de composés de formule (X) avec des acides boroniques de formule (XI) selon le Schéma 9. Les conditions de réaction préférées comprennent une réaction avec de l'acétate de cuivre et de la triéthylamine.

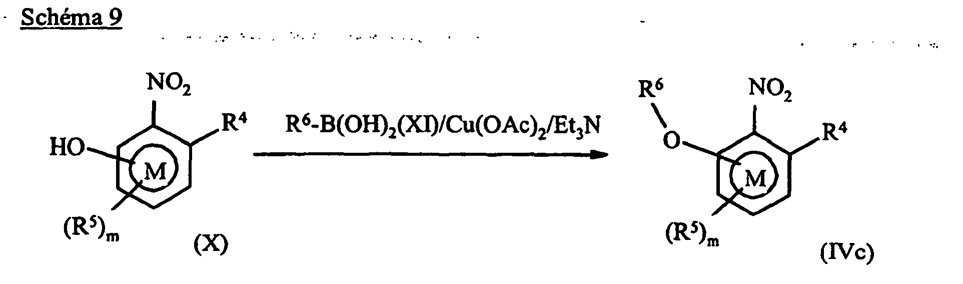

Les composés de formule (IVd), c'est-à-dire les composés de formule (IV) dans laquelle A représente une liaison directe peuvent être préparés selon le Schéma 10 à partir de composés de formule (XII) dans laquelle X^{Z} représente un groupe partant, de préférence un atome d'halogènes.

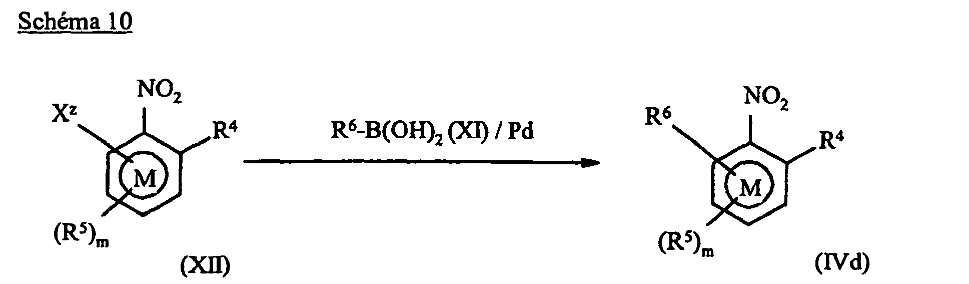

Les composés de formule (IV) dans laquelle A représente une liaison directe et R⁶ représente un radical hétérocyclyle peuvent être préparés en utilisant de nombreuses méthodes connues de l'homme du métier (par exemple voir "Comprehensive Heterocyclic Chemistry", Vols 1-7, A. R. Katritzky et C. W. Rees). À titre d'exemple, les voies d'accès aux composés de formule (IV) contenant un radical 1,2,4-oxadiazol-3-yl [composé (IVe)] et un radical 1,3,4-oxadiazol-2-yl [composé (IVf)] sont présentées dans les Schémas 11 et 12.

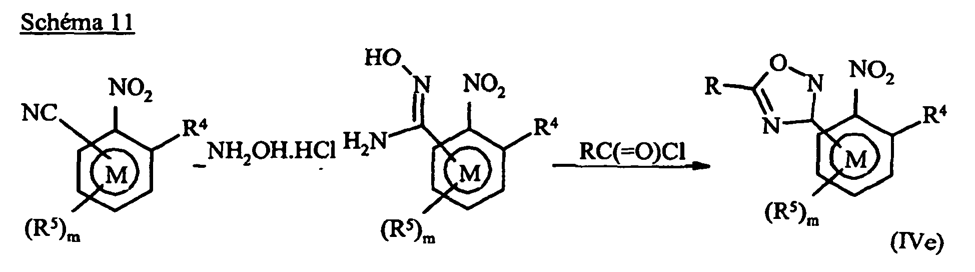

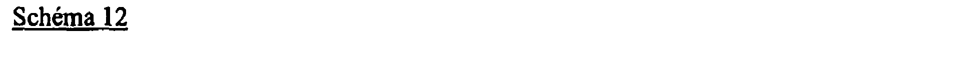

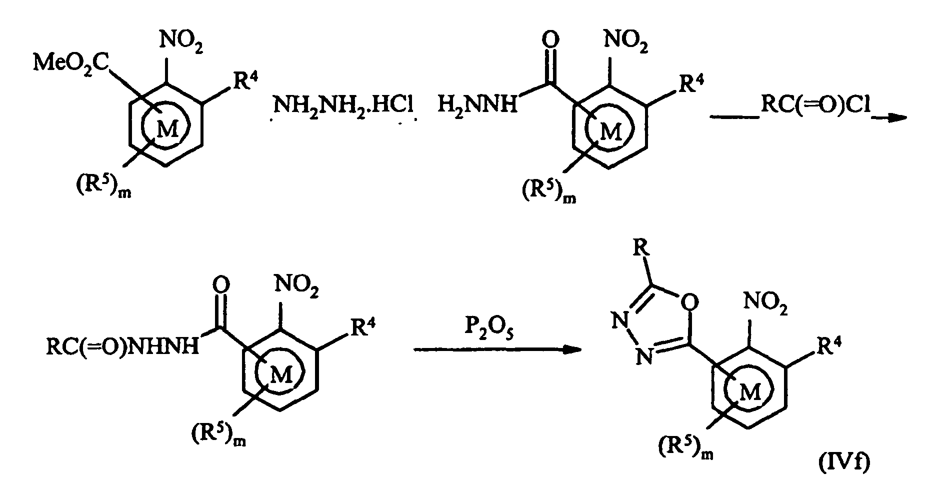

Les composés de formule (IIIf), c'est-à-dire les composés de formule générale (III) dans laquelle A représente -CHR⁷- avec R⁷ représentant un radical hydroxy ou alkoxy, peuvent être préparés à partir de composés de formule (IIIg) selon la méthodologie décrite dans le Schéma 13, où R représente un radical alkyle éventuellement substitué ou l'atome d'hydrogène, et où R⁶ représente un radical carbo- ou hétéro-cyclique éventuellement substitué et pouvant former un anion dans des conditions basiques.

De manière analogue, la suite réactionnelle décrite dans le Schéma 13 peut être effectuée sur les composés de formule (IIIf) où la fonction amine est remplacée par un précurseur de la dite fonction, par exemple le radical -NO₂ ou le radical -N(CW)R₀.

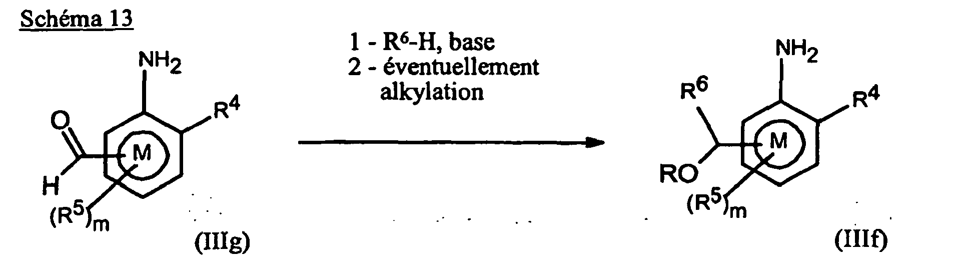

De manière alternative, en utilisant des chimies similaires à celles décrites précédemment, les composés de formule générale (I) peuvent être préparés en introduisant R⁶ après la formation de l'entité -NR₀(CW)Y.

D'autres méthodes pour accéder aux composés de formule (I) ainsi qu'à leurs intermédiaires et composés de départ apparaîtront évidentes à l'homme de l'art, comme celles décrites par exemple dans Brown, B.R. *The Organic Chemistry of Aliphatic Nitrogen Compounds* (Oxford Science Publications, **1994)** et Patai, S. *"The chemistry of cyanates and their thio derivatives*" (John Wiley & Sons, **1977).**

De plus, les composés selon l'invention peuvent être préparés en utilisant les techniques bien connues de chimie combinatoire.

L'invention est illustrée par les Exemples suivants mais ne doit pas être comprise comme limitée par ceux-ci. Les structures des composés nouveaux, isolés, ont été confirmées par R.M.N. et/ou d'autres analyses appropriées. Les spectres de R.M.N. du proton (¹H R.M.N.) ont été enregistrés dans du deuterochloroforme et les déplacements chimiques (δ) sont donnés en parties par million par rapport au tétraméthylsilane.

### Exemple 1

### N'-(4-{[3-(1,1-diméthyléthyl)phényl]oxy}-2,5-diméthylphényl)-N-éthyl-N-méthylthiourée (Composé 27)

À une solution de 1-{[3-(1,1-diméthyléthyl)phényl]oxy}-4-isothiocyanato-2,5-diméthylbenzène (1,9 g) dans du tétrahydrofurane (6 mL) à 0°C est ajouté goutte à goutte de l'éthylméthylamine (450 mg). Le mélange réactionnel obtenu est agité à température ambiante pendant 1h puis concentré et trituré par de l'heptane. Le produit du titre est obtenu sous forme d'un précipité banc.

### Préparation des Produits de Départ

### 1-chloro-2,5-diméthyl-4-nitrobenzène

À un mélange refroidit à 10°C de 2-chloro-1,4-xylène (1,4 g), d'acide acétique (17 mL) et d'acide sulfurique à 98% (1 mL) est ajouté goutte à goutte un mélange sulfonitrique à 0°C composé d'acide sulfurique (3,8 mL) et d'acide nitrique (1 mL). Le milieu réactionnel est versé dans un mélange eau/glace puis filtré. Le précipité jaune est recristallisé dans de l'éther de pétrole pour donner le composé du titre.

### 1- {[3-(1,1-diméthyléthyl)phényl]oxy}-2,5-diméthyl-4-nitrobenzène

À une solution de 1-chloro-2,5-diméthyl-4-nitrobenzène (9,5 g) dans du diméthylformamide (60 mL) sous atmosphère inerte est ajouté du 3-t-butylphénol (7,5 g) et du carbonate de potassium (6,9 g). Le mélange réactionnel obtenu est chauffé à reflux durant 5h, puis partagé dans un mélange eau/éther diisopropylique. La phase organique est lavée par une solution aqueuse basique, puis par de l'eau à pH 7. Un séchage sur sulfate de magnésium puis un ajout de noir de charbon suivi d'une filtration sur célite permet d'obtenir après concentration le produit du titre.

### 4-{[3-(1,1-diméthyléthyl)phényl]oxy}-2,5-diméthylamiline

À un mélange de 1-{[3-(1,1-diméthyléthyl)phényl]oxy}-2,5-diméthyl-4-nitrobenzène (8,65 g) et de palladium 5% sur charbon (100 mg) dans du *n*-propanol (60 mL) chauffé à 90°C est ajouté goutte à goutte de l'hydrate d'hydrazine (4,3 mL). Le mélange réactionnel est maintenu à 90°C pendant 2h puis filtré sur célite. Une concentration permet d'obtenir le composé du titre.

### 1-{[3-(1,1-diméthyléthyl)phényl]oxy}-4-isothiocyanato-2,5-diméthylbenzène

À un mélange biphasique à température ambiante de 4-((3-(1,1-diméthyléthyl)phényl)oxy)-2,5-diméthylaniline (1,35 g) dans du toluène (6 mL) et de bicarbonate de sodium (840 mg) dans de l'eau (6 mL) est ajouté du thiophosgène (0,38 mL). Le mélange réactionnel obtenu est agité durant 2h puis décanté. La phase aqueuse est extraite par du toluène, puis les phases organiques réunifiées sont lavées par de l'eau, séchées (sulfate de magnésium) puis concentrées pour donner le produit du titre.

La procédure décrite pour la synthèse de la *N'*-(4-((3-(1,1-diméthyléthyl)phényl)oxy)-2,5-diméthylphényl)-*N*-éthyl-*N*-méthylthiourée a été généralisée à d'autres amines et transposée à d'autres conditions de température et de solvants, notamment l'acétonitrile, le diéthyléther et le diméthylformamide. Cette procédure a été également utilisée pour la synthèse des thiosemicarbazides et des thiohydroxylurées.

Dans le cas de la 1-(4-{[3-(1,1-diméthyléthyl)phényl]oxy}-2,5-diméthylphényl)-1,3-dihydro-2H-imidazole-2-thione (Composé 16), la thiourée cyclique a été obtenue en faisant réagir selon la procédure décrite précédemment le diméthyl acétal d'aminoacétaldéhyde sur la 4-{[3-(1,1-diméthyléthyl)phényl]oxy}-2,5-diméthylaniline.

La procédure décrite pour préparer la 4- {[3-(1,1-diméthyléthyl)phényl]oxy}-2,5-diméthylaniline a également été utilisée pour préparer les anilines suivantes: la 4-[(3-chlorophényl)oxy]-2,5-diméthylaniline, la 4-{[4-chloro-3-(trifluorométhyl)phényl]-oxy}-2,5-diméthylaniline, la 4-{[4-fluoro-3-(trifluorométhyl)phényl]oxy}-2,5-diméthylaniline, la 2,5-diméthyl-4-{[3-(trifluorométhyl)phényl]oxy}aniline, la 4-[(4-éthylphényl)oxy]-2,5-diméthylaniline, la 4-[(4-chlorophényl)oxy]-2,5-diméthylaniline et la 4-{[3-(1,1-diméthyléthyl)phényl]oxy}-2-(trifluorométhyl)aniline.

D'autres anilines utilisées comme intermédiaires dans la synthèse de produits exemplifiés ont été synthétisées selon les méthodes suivantes.

### Préparation de la 4-(5-(1.1-diméthyléthyl)-1,3,4-oxadiazol-2-yl)-2-méthylaniline

À une solution à température ambiante de 3-méthyl-4-nitrobenzoate de méthyle (25 g) dans du méthanol (300 mL) est ajoutée de l'hydrate d'hydrazine (6,7 mL). Le mélange réactionnel est chauffé pendant 15 minutes au reflux puis refroidit. Le précipité filtré et séché correspond au 3-méthyl-4-nitrobenzohydrazide.

À un mélange constitué de 3-méthyl-4-nitrobenzohydrazide (3,9 g), de triéthylamine (3 mL) et de chlorure d'acide 2,2-diméthylpropanoïque (2,6 mL) dans du dichlorométhane (120 mL) et agité pendant 2h est ajoutée de l'eau. Après séchage, évaporation et tritutation de la phase organique dans de l'éther de pétrole, le *N'*-(2,2-diméthylpropanoyl)-3-méthyl-4-nitrobenzohydrazide est obtenu sous forme de précipité.

À une suspension de *N'*-(2,2-diméthylpropanoyl)-3-méthyl-4-nitrobenzohydrazide (5,1 g) dans du toluène (200 mL) est ajouté du pentoxide de phosphore (10 g). Après chauffage à reflux pendant 2h le mélange réactionnel est versé dans de l'eau glacée et extrait par du diéthyléther. Les phases organiques sont combinées, séchées sur du sulfate de magnésium, évaporées et purifiées par chromatographie sur silice (éluant diéthyléther/éther de pétrole : 2:1) pour donner le 2-(1,1-diméthyléthyl)-5-(3-méthyl-4-nitrophényl)-1,3,4-oxadiazole.

Un mélange de 2-(1,1-diméthyléthyl)-5-(3-méthyl-4-nitrophényl)-1,3,4-oxadiazole (1,6 g), de FeCl₃.6H₂O (5 g) et de poudre de zinc (4 g) dans un mélange diméthylformamide/eau (1:1, 25 mL) est chauffé à 100°C pendant 30 minutes puis filtré sur célite. Après dilution avec une solution aqueuse saturée en carbonate de sodium, le mélange réactionnel est extrait par du diéthyléther. Un séchage sur sulfate de magnésium et une évaporation de la phase organique permet d'obtenir l'aniline du titre.

### Préparation de la 2,5-diméthyl-4-[(3-phényl-1,2,4-thiadiazol-5-yl)oxy]phénylamine

À une suspension d'hydrure de sodium (dispersion à 60% dans de l'huile minérale, 3g) dans du diméthylformamide anhydre (300 mL) est ajouté à température ambiante du 4-amino-2,5-diméthylphénol (10,5 g). Le mélange réactionnel est agité durant 30 minutes puis du 5-bromo-3-phényl-1,2,4-thiadiazole (18 g) préparé selon *Chem*. *Ber*. **1961,** *94,* 2043 est ajouté lentement. Après agitation durant 15h, le mélange réactionnel est dilué dans de l'eau glacée puis extrait par du diéthyléther. Un séchage sur sulfate de magnésium suivi d'une concentration des phases organiques réunies permet d'obtenir le composé du titre.

### Préparation de la 4-[3 4-dihydro-2(1H)-isoquinoléinyl]-2,5-diméthylaniline

Un mélange de 1-chloro-2,5-diméthyl-4-nitrobenzène (1,65 g) et de 1,2,3,4-tétrahydroisoquinoléine (5 mL) dans de la *N*-méthylpyrolidine (5 mL) est agité à 140°C pendant 15h. Une dilution dans de l'eau suivie d'une extraction au diéthyléther et d'une chromatographie sur silice (éluant éther de pétrole/diéthyléther : 1:1) de la phase organique séchée et évaporée permet d'obtenir la 2-(2,5-diméthyl-4-nitrophényl)-1,2,3,4-tétrahydro-isoquinoléine.
La 2-(2,5-diméthyl-4-nitrophényl)-1,2,3,4-tétrahydro-isoquinoléine est réduite selon les méthodes exemplifiées précédemment pour donner l'aniline du titre.

### Préparation de la 4-(1-benzothiophén-2-yl)-2,5-diméthylaniline

Un mélange de 1-bromo-2,5-diméthyl-4-nitrobenzène (3,6 g), d'acide (1-benzothién-2-yl)boronique (3,6 g), de palladium tétrakistriphénylphosphine (0,4 g), de solution aqueuse (2M) de carbonate de sodium (17 mL), dans de l'éthanol (36 mL) et du toluène (100 mL) est agité au reflux durant 18h. Une évaporation partielle suivie d'une extraction à l'acétate d'éthyle, d'un séchage de la phase organique suivi d'une nouvelle évaporation permet d'obtenir le 2-(2,5-diméthyl-4-nitrophényl)-1-benzothiophène.
Le 2-(2,5-diméthyl-4-nitrophényl)-1-benzothiophène est réduit selon les méthodes décrites précédemment pour donner l'aniline du titre.

### Exemple 2

### 3-[4-(1-benzothién-2-yl)-2,5-diméthylphényl]-2-thioxo-1,3-thiazolidin-4-one (Composé 3)

Une solution de 2-(4-isothiocyanato-2,5-diméthylphényl)-1-benzothiophène (0,22 g) et de sulfénylacétate d'éthyle (0.09 g) dans du diéthyléther (2 mL) est agitée pendant 18h à température ambiante. Le milieu réactionnel est évaporé puis trituré dans de l'éther de pétrole pour donner le composé du titre.

### Exemple 3

### N-(4-{[4-chloro-3-(trifluorométhyl)phényl]oxy}-2,5-diméthylphényl)-N, N'-diéthyl-N'-méthylurée (Composé 116)

À une solution de 4-{[4-chloro-3-(trifluorométhyl)phényl]oxy}-2,5-diméthylaniline (3 g) dans de l'acétate d'éthyle anhydre (50 mL) est ajouté à 60 °C sous argon une spatule de charbon actif puis du diphosgène (2,07 g). Le mélange réactionnel obtenu est chauffé à 60 °C pendant 1h puis agité à température ambiante pendant 15h. Une filtration sur célite suivie d'une évaporation et d'une reprise dans de l'éther de pétrole (50 mL) auquel est ajouté de la *N*-éthylméthylamine (1,12 g) permet d'obtenir après évaporation la *N'*-(4-{[4-chloro-3-(trifluorométhyl)phényl]oxy}-2,5-diméthylphényl)-*N*-éthyl-*N*-méthylurée. À une solution de ce dernier composé (380 mg) dans du tétrahydrofurane (2 mL) est ajouté une suspension d'hydrure de sodium à 60% dans de l'huile minérale (46 mg) puis de l'iodure d'éthyle (300 mg). Le mélange réactionnel est agité à 50°C pendant 2h puis dilué dans de l'eau et extrait avec du diéthyléther. Un séchage de la phase organique sur du sulfate de magnésium suivi d'une évaporation permet d'obtenir le composé du titre.

La procédure décrite pour la synthèse de la *N*'-(4-{[4-chloro-3-(trifluorométhyl)phényl]oxy}-2,5-diméthylphényl)-*N*-éthyl-*N*-méthylurée a été généralisée à d'autres amines et transposée à d'autres solvants, notamment l'acétonitrile, le diéthyléther et le diméthylformamide. Des procédures similaires ont été utilisées pour la synthèse des hydroxylurées, des semicarbazides et des carbamates.

Les exemples suivants (Tableau 1) illustrent, de manière non limitative quelques composés selon la présente invention et qui ont été synthétisés à partir des procédures précédentes

**Tableau 1**

| | | | | | | | |
|---|---|---|---|---|---|---|---|
| 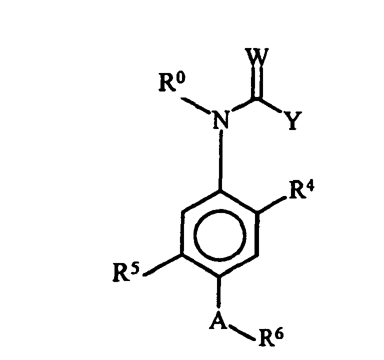 | | | | | | | |
| N.B. : Me signifie méthyle, Et signifie éthyle, Pr signifie propyle, Bu signifie butyle, Ph signifie phényle, *n* signifie linéaire, *i* signifie *iso, t* signifie *tertio.* Par souci de clarté, les indices n'ont pas été représentés ; ainsi CH2 signifie CH₂, CO2 signifie CO₂, etc. | | | | | | | |

| **Cmpsé** | **R5** | **R4** | **W** | **R0** | **Y** | **A** | **R6** |
|---|---|---|---|---|---|---|---|
| 1 | Me | Me | S | H | NEtMe | liaison directe | 2-benzothiophényl |
| 2 | Me | Me | S | H | NMe2 | liaison directe | 2-benzothiophényl |
| 3 | Me | Me | S | liaison simple avec | 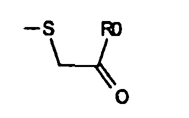 | liaison directe | 2-benzothiophényl |
| 4 | Me | Me | S | H | N-Morpholino | liaison directe | 2-benzothiophényl |
| 5 | Me | Me | S | H | NHCH2Ph | liaison directe | 2-benzothiophényl |
| 6 | Me | Me | O | H | OEt | O | 3-tBu-phényl |
| 7 | Me | Me | S | liaison simple avec Y | 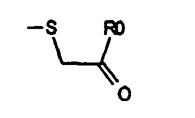 | O | 3-trifluorométhylphényl |
| 8 | H | Me | S | H | NHMe | liaison directe | 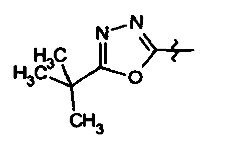 |
| 9 | H | Me | S | H | NH-cyclopropyle | liaison directe | 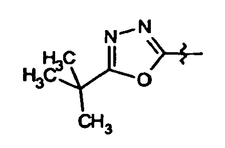 |
| 10 | H | Me | S | H | NHCH2Ph | liaison directe | 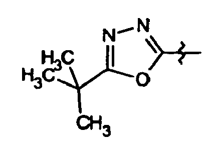 |
| 11 | H | Me | S | H | N-Morpholino | liaison directe | 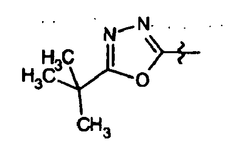 |
| 12 | Me | Me | O | H | OMe | liaison directe | 2-benzothiophényl |
| 13 | Me | Me | S | H | NHMe | liaison directe | 2-benzothiophényl |
| 14 | M | Me | O | H | O-*t*butyle | liaison directe | 2-benzothiophényl |
| 15 | H | Me | S | H | NHMe | liaison directe | 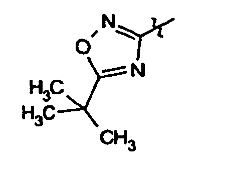 |
| 16 | Me | Me | S | liaison simple avec Y liaison simple avec Y | 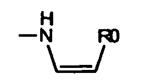 | O | 3-tBu-phényl |
| 17 | Me | Me | S | H | 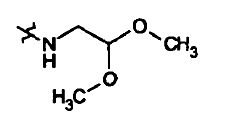 | O | 3-tBu-phényl |
| 18 | Me | Me | S | H | 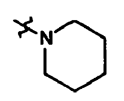 | O | 3-tBu-phényl |
| 19 | Me | Me | S | H | NHEt | O | 3-tBu-phényl |
| 20 | Me | Me | S | H | N-Morpholino | O | 3-tBu-phényl |
| 21 | Me | Me | S | H | NH*i*Pr | O | 3-tBu-phényl |
| 22 | Me | Me | S | H | NH*n* Pr | O | 3-tBu-phényl |
| 23 | Me | Me | S | H | NHcyclopropyle | O | 3-tBu-phényl |
| 24 | Me | Me | S | H | NMe2 | O | 3-tBu-phényl |
| 25 | Me | Me | S | H | 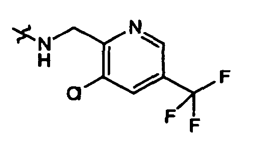 | O | 3-tBu-phényl |
| 26 | Me | Me | S | H | 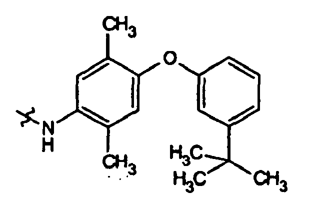 | O | 3-tBu-phényl |
| 27 | Me | Me | S | H | NEtMe | O | 3-tBu-phényl |
| 28 | Me | Me | S | H | Nn BuMe | O | 3-tBu-phényl |
| 29 | Me | Me | S | H | 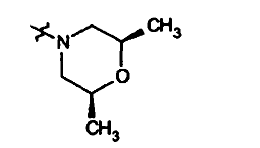 | O | 3-tBu-phényl |
| 30 | Me | Me | S | H | NHMe | O | 4-Et-phényl |
| 31 | Me | Me | S | H | 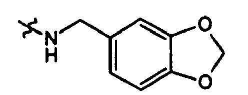 | O | 3-tBu-phényl |
| 32 | Me | Me | S | H | 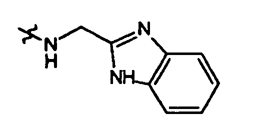 | O | 3-tBu-phényl |
| 33 | Me | Me | O | H | O-*t*butyle | O | 3-tBu-phényl |
| 34 | Me | Me | S | H | 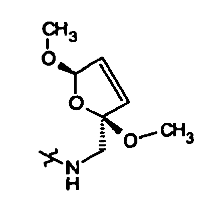 | O | 3-tBu-phényl |
| 35 | Me | Me | S | H | 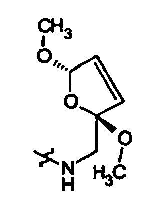 | O | 3-tBu-phényl |
| 36 | Me | Me | S | H | 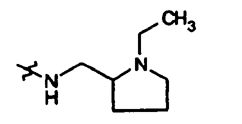 | O | 3-tBu-phényl |
| 37 | Me | Me | S | H | NHMe | O | 4-chlorophényl |
| 38 | Me | Me | O | H | NHiPr | O | 3-tBu-phényl |
| 39 | Me | Me | O | Me | O-*t*butyle | O | 3-tBu-phényl |
| 40 | Me | Me | S | H | 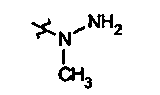 | O | 3-tBu-phényl |
| 41 | Me | Me | S | H | 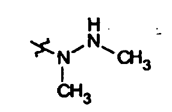 | O | 3-tBu-phényl |
| 42 | Me | Me | S | H | NHMe | O | 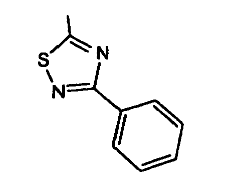 |
| 43 | M | Me | S | | NEtMe | O | 3-trifluorométhylphényl |
| 44 | M | Me | S | liaison simple avec Y | 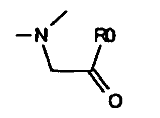 | O | 3-tBu-phényl |
| 45 | Me | Me | S | liaison simple avec Y | 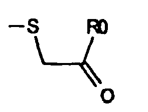 | O | 3-tBu-phényl |
| 46 | Me | Me | S | liaison simple avec Y | 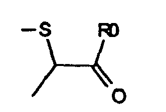 | O | 3-tBu-phényl |
| 47 | H | CF3 | S | H | NEtMe | O | 3-tBu-phényl |
| 48 | Me | Me | S | liaison simple avec Y | 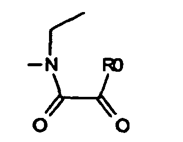 | O | 3-tBu-phényl |
| 49 | Me | Me | O | H | OMe | O | 3-trifluorométhylphényl |
| 50 | Me | Me | S | H | NHMe | O | 3-tBu-phényl |
| 51 | Me | Me | S | H | NHMe | liaison directe | 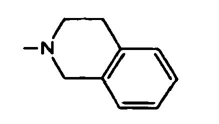 |
| 52 | Me | Me | S | liaison simple avec Y | 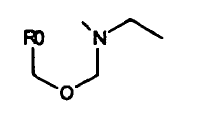 | O | 3-tBu-phényl |
| 53 | Me | Me | S | liaison simple avec Y | 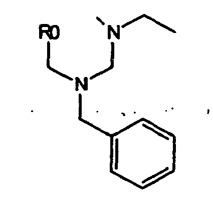 | O | 3-tBu-phényl |
| 54 | Me | Me | S | liaison simple avec Y | 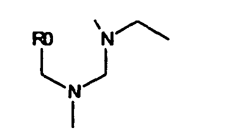 | O | 3-tBu-phényl |
| 55 | Me | Me | S | liaison simple avec Y | 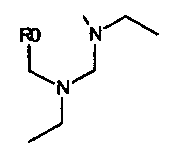 | O | 3-tBu-phényl |
| 56 | Me | Me | S | liaison simple avec Y | 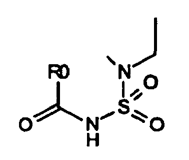 | O | 3-tBu-phényl |
| 57 | Me | Me | O | H | NHPh | O | 3-trifluorométhylphényl |
| 58 | Me | Me | S | H | NHPh | O | 3-trifluorométhylphényl |
| 59 | Me | Me | S | H | 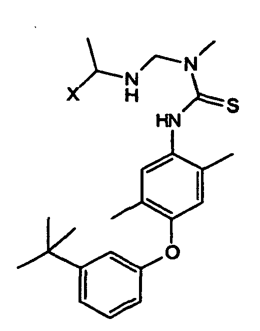 | O | 3-tBu-phényl |
| 60 | Me | Me | O | H | N-Morpholino | O | 3-tBu-phényl |
| 61 | Me | Me | O | H | NEtMe | O | 3-tBu-phényl |
| 62 | Me | Me | O | H | 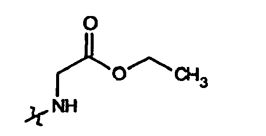 | O | 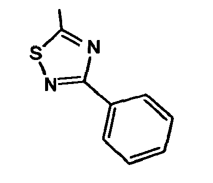 |
| 63 | Me | Me | O | liaison simple avec Y | 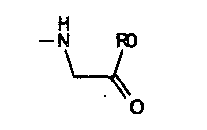 | O | 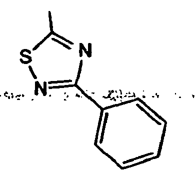 |
| 64 | Me | Me | S | H | NHPh | O | 3-tBu-phényl |
| 65 | Me | Me | O | H | NEtMe | O | 4-chloro-3-trifluorométhylphényl |
| 66 | Me | Me | O | H | 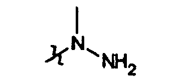 | O | 4-chloro-3-trifluorométhylphényl |
| 67 | Me | Me | O | H | 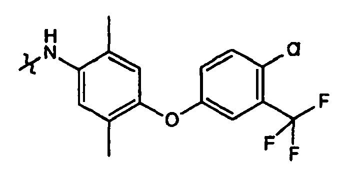 | O | 4-chloro-3-trifluorométhylphényl |
| 68 | Me | Me | O | H | 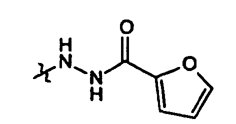 | O | 4-chloro-3-trifluorométhylphényl |
| 69 | Me | Me | O | H | 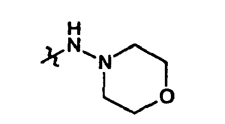 | O | 4-chloro-3-trifluorométhylphényl |
| 70 | Me | Me | O | H | 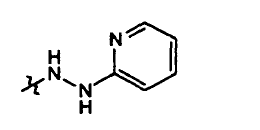 | O | 4-chloro-3-trifluorométhylphényl |
| 71 | Me | Me | O | H | 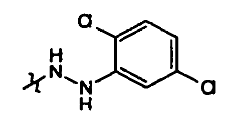 | O | 4-chloro-3-trifluorométhylphényl |
| 72 | Me | Me | O | H | 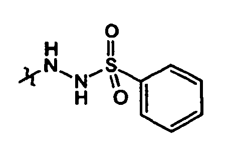 | O | 4-chloro-3-trifluorométhylphényl |
| 73 | Me | Me | O | H | 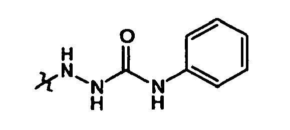 | O | 4-chloro-3-trifluorométhylphényl |
| 74 | Me | Me | O | H | 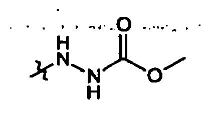 | O | 4-chloro-3-trifluorométhylphényl |
| 75 | Me | Me | O | H | 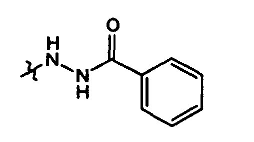 | O | 4-chloro-3-trifluorométhylphényl |
| 76 | Me | Me | O | H | 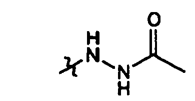 | O | 4-chloro-3-trifluorométhylphényl |
| 77 | Me | Me | O | H | 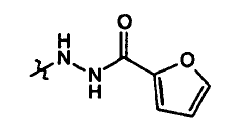 | O | 4-fluoro-3-trifluorométhylphényl |
| 78 | Me | Me | O | H | 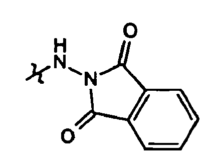 | O | 4-fluoro-3-trifluorométhylphényl |
| 79 | Me | Me | O | H | 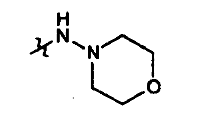 | O | 4-fluoro-3-trifluorométhylphényl |
| 80 | Me | Me | O | H | 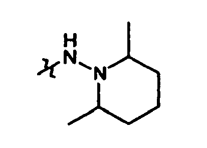 | O | 4-fluoro-3-trifluorométhylphényl |
| 81 | Me | Me | O | H | 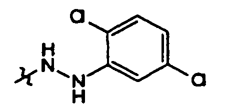 | O | 4-fluoro-3-trifluorométhylphényl |
| 82 | Me | Me | O | H | 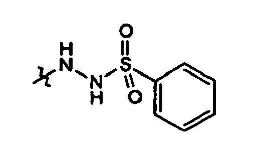 | O | 4-fluoro-3-trifluorométhylphényl |
| 83 | Me | Me | O | H | 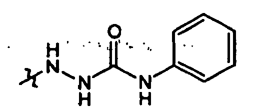 | O | 4-fluoro-3-trifluorométhylphényl |
| 84 | Me | Me | O | H | 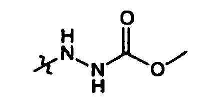 | O | 4-fluoro-3-trifluorométhylphényl |
| 85 | Me | Me | O | H | 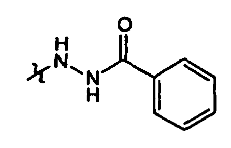 | O | 4-fluoro-3-trifluorométhylphényl |
| 86 | Me | Me | O | H | 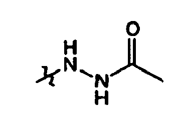 | O | 4-fluoro-3-trifluorométhylphényl |
| 87 | Me | Me | O | H | 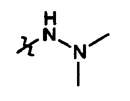 | O | 4-fluoro-3-trifluorométhylphényl |
| 88 | Me | Me | O | H | 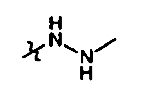 | O | 4-fluoro-3-trifluorométhylphényl |
| 89 | Me | Me | O | H | NHEt | O | 4-chloro-3-trifluorométhylphényl |
| 90 | Me | Me | O | H | NHiPr | O | 4-chloro-3-trifluorométhylphényl |
| 91 | Me | Me | O | H | NHcyclopropyl | O | 4-chloro-3-trifluorométhylphényl |
| 92 | Me | Me | O | H | 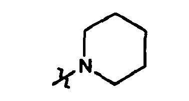 | O | 4-chloro-3-trifluorométhylphényl |
| 93 | Me | Me | O | H | 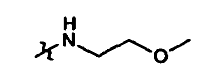 | O | 4-chloro-3-trifluorométhylphényl |
| 94 | Me | Me | O | H | NHPh | O | 4-chloro-3-trifluorométhylphényl |
| 95 | Me | Me | O | H | NHCH2Ph | O | 4-chloro-3-trifluorométhylphényl |
| 96 | Me | Me | O | H | 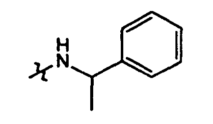 | O | 4-chloro-3-trifluorométhylphényl |
| 97 | Me | Me | O | H | 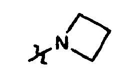 | O | 4-chloro-3-trifluorométhytphényl |
| 98 | Me | Me | O | H | N-Morpholino | O | 4-chloro-3-trifluorométhylphényl |
| 99 | Me | Me | O | H | 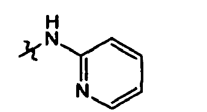 | O | 4-chloro-3-trifluorométhylphényl |
| 100 | Me | Me | O | H | 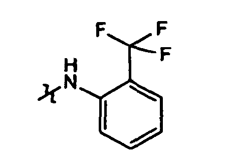 | O | 4-chloro-3-trifluorométhylphényl |
| 101 | Me | Me | O | H | 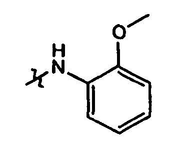 | O | 4-chloro-3-trifluorométhylphényl |
| 102 | Me | Me | O | H | 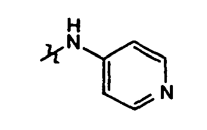 | O | 4-chloro-3-trifluorométhylphényl |
| 103 | Me | Me | O | H | 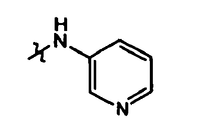 | O | 4-chloro-3-trifluorométhylphényl |
| 104 | Me | Me | O | H | 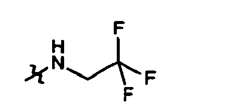 | O | 4-chloro-3-trifluorométhylphényl |
| 105 | Me | Me | O | H | NMet Bu | O | 4-chloro-3-trifluorométhylphényl |
| 106 | Me | Me | O | H | 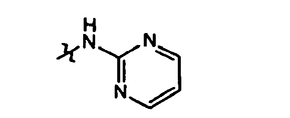 | O | 4-chloro-3-trifluorométhylphényl |
| 107 | Me | Me | O | H | OMe | O | 4-chloro-3-trifluorométhylphényl |
| 108 | Me | Me | O | H | OEt | O | 4-chloro-3-trifluorométhylphényl |
| 109 | Me | Me | O | H | Oi-Pr | O | 4-chloro-3-trifluorométhylphényl |
| 110 | Me | Me | O | H | OCH2Ph | O | 4-chloro-3-trifluorométhylphényl |
| 111 | Me | Me | O | H | 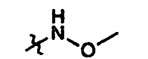 | O | 4-chloro-3-trifluorométhylphényl |
| 112 | Me | Me | O | H | 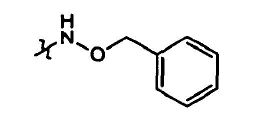 | O | 4-chloro-3-trifluorométhylphényl |
| 113 | Me | Me | O | H | 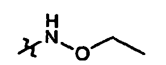 | O | 4-chloro-3-trifluorométhylphényl |
| 114 | Me | Me | O | H | 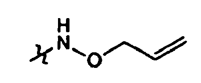 | O | 4-chloro-3-trifluorométhylphényl |
| 115 | Me | Me | O | H | 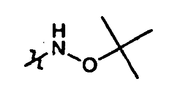 | O | 4-chloro-3-trifluorométhylphényl |
| 116 | Me | Me | O | Et | NEtMe | O | 4-chloro-3-trifluorométhylphényl |
| 117 | Me | Me | O | Benzyl | NEtMe | O | 4-chloro-3-trifluorométhylphényl |
| 118 | Me | Me | O | Me | NEtMe | O | 4-chloro-3-trifluorométhylphényl |
| 119 | Me | Me | O | 3-propar gyl | NEtMe | O | 4-chloro-3-trifluorométhylphényl |
| 120 | Me | Me | O | 3-allyl | NEtMe | O | 4-chloro-3-trifluorométhylphényl |
| 121 | Me | Me | O | (alphaméthyl) benzyl | NEtMe | O | 4-chloro-3-trifluorométhylphényl |
| 122 | Me | Me | S | H | 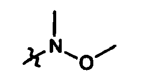 | O | 3-*t*butylphényl |
| 123 | Me | Me | S | H | N-thiazolidinyl | O | 3-*t*butylphényl |
| 124 | Me | Me | S | H | NMe*i*Pr | O | 3-*t*butylphényl |
| 125 | Me | Me | S | H | NEt2 | O | 3-*t*butylphényl |
| 126 | Me | Me | S | H | NHCH2Ph | O | 3-*t*butylphényl |
| 127 | Me | Me | S | H | 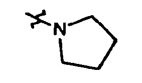 | O | 3-*t*butylphényl |
| 128 | Me | Me | S | H | 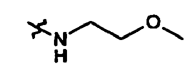 | O | 3-*t*butylphényl |
| 129 | Me | Me | S | H | 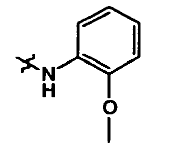 | O | 3-*t*butylphényl |
| 130 | Me | Me | S | H | 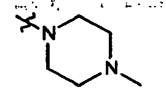 | O | 3-*t*butylphényl |
| 131 | Me | Me | S | H | 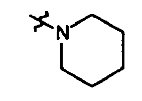 | O | 4-chloro-3-trifluorométhylphényl |
| 132 | Me | Me | S | H | N-Morpholino | O | 4-chloro-3-trifluorométhylphényl |
| 133 | Me | Me | S | H | NEt2 | O | 4-chloro-3-trifluorométhylphényl |
| 134 | Me | | S | H | NHCH2Ph | O | 4-chloro-3-trifluorométhylphényl |
| 135 | Me | Me | S | H | 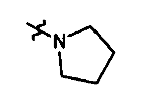 | O | 4-chloro-3-trifluorométhylphényl |
| 136 | Me | Me | S | H | NHcyclopropyl | O | 4-chloro-3-trifluorométhylphényl |
| 137 | Me | Me | S | H | 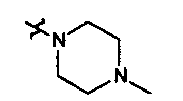 | O | 4-chloro-3-trifluorométhylphényl |
| 138 | Me | Me | S | H | 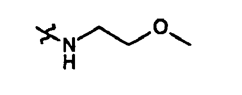 | O | 4-chloro-3-trifluorométhylphényl |
| 139 | Me | Me | S | H | 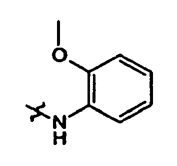 | O | 4-chloro-3-trifluorométhylphényl |
| 140 | Me | Me | S | H | 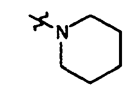 | O | 4-fluoro-3-trifluorométhylphényl |
| 141 | Me | Me | S | H | N-Morpholino | O | 4-fluoro-3-trifluorométhylphényl |
| 142 | Me | Me | S | H | NEt2 | O | 4-fluoro-3-trifluorométhylphényl |
| 143 | Me | Me | S | H | NHCH2Ph | O | 4-fluoro-3-trifluorométhylphényl |
| 144 | Me | Me | S | H | 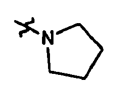 | O | 4-fluoro-3-trifluorométhylphényl |
| 145 | Me | Me | S | H | NHcyclopropyl | O | 4-fluoro-3-trifluorométhylphényl |
| 146 | Me | Me | S | H | 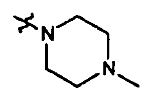 | O | 4-fluoro-3-trifluorométhylphényl |
| 147 | Me | Me | S | H | NH*i*Pr | O | 4-fluoro-3-trifluorométhylphényl |
| 148 | Me | Me | S | H | 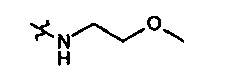 | O | 4-fluoro-3-trifluorométhylphényl |
| 149. | Me | Me | S | H | NEtMe | O | 4-fluoro-3-trifluorométhylphényl |
| 150 | Me | Me | S | H | 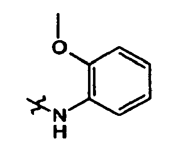 | O | 4-fluoro-3-trifluorométhylphényl |
| 151 | Me | Me | S | H | 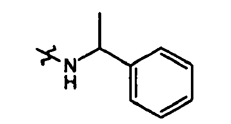 | O | 4-fluoro-3-trifluorométhylphényl |
| 152 | Me | Me | S | H | 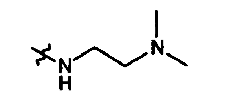 | O | 3-*t*butylphényl |
| 153 | Me | Me | S | H | NH*t* Bu | O | 4-chloro-3-trifluorométhylphényl |
| 154 | Me | Me | S | H | NH*t* Bu | O | 4-fluoro-3-trifluorométhylphényl |
| 155 | Me | Me | S | H | NH*t* Bu | O | 3-*t*butylphényl |
| 156 | Me | Me | S | H | NMe2 | O | 4-chloro-3-trifluorométhylphényl |
| 157 | Me | Me | S | H | NMe2 | O | 4-fluoro-3-trifluorométhylphényl |
| 158 | Me | Me | S | H | NHEt | O | 4-chloro-3-trifluorométhylphényl |
| 159 | Me | Me | S | H | NH2 | O | 3-*t*butylphényl |
| 160 | Me | Me | S | H | NH2 | O | 4-chloro-3-trifluorométhylphényl |
| 161 | Me | Me | S | H | 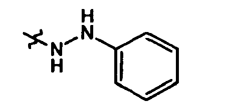 | O | 3-*t*butylphényl |
| 162 | Me | Me | S | H | 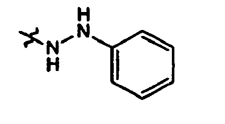 | O | 4-chloro-3-trifluorométhylphényl |
| 163 | Me | Me | S | H | 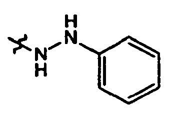 | O | 4-fluoro-3-trifluorométhylphényl |
| 164 | Me | Me | S | H | 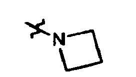 | O | 3-*t*butylphényl |
| 165 | Me | Me | S | H | 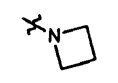 | O | 4-chloro-3-trifluorométhylphényl |
| 166 | Me | Me | S | H | 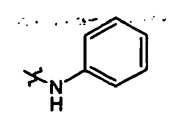 | O | 4-chloro-3-trifluorométhylphényl |
| 167 | Me | Me | S | H | 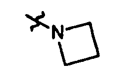 | O | 4-fluoro-3-trifluorométhylphényl |
| 168 | Me | Me | S | H | 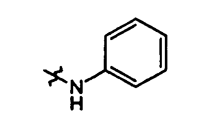 | O | 4-fluoro-3-trifluorométhylphényl |
| 169 | Me | Me | S | H | 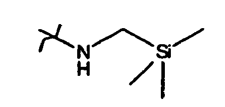 | O | 4-chloro-3-trifluorométhylphényl |
| 170 | Me | Me | S | H | 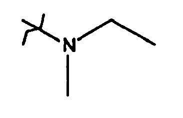 | O | 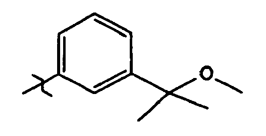 |
| 171 | Me | Me | S | H | 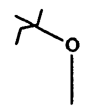 | O | 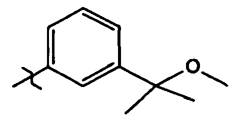 |

Les caractéristiques analytiques des composés du Tableau 1 sont décrites dans le tableau suivant.

**Tableau 2**

| | | | |
|---|---|---|---|
| *NB:* la colonne (M+1) représente le (pic moléculaire + 1) tel que déterminé expérimentalement par la technique de spectrométrie de masse API+ | | | |

| **Cmpsé** | **p.f. (°C)** | **NMR (CDCl₃)** | **(M+1)** |
|---|---|---|---|
| 1 | 135 | | |
| 2 | 183 | | |
| 3 | | | 370 |
| 4 | 177 | | |
| 5 | 93 | | |
| 6 | | | 342 |
| 7 | | | 398 |
| 8 | 201 | | |
| 9 | 156 | | |
| 10 | 133 | | |
| 11 | 167 | | |
| 12 | 157 | | |
| 13 | | | 327 |
| 14 | 133 | | |
| 15 | 186 | | |
| 16 | | 1,30 (s,9H,(CH₃)₃) ; 2,05 (s,3H,ArCH₃) ; 2,30 (s,3H,ArCH₃) | |
| 17 | | 1,30 (s,9H,(CH₃)₃) ; 2,15 (s,3H,ArCH₃) ; 2,20 (s,3H,ArCH₃) ; 3,35 (s,3H,OCH₃) ; 4,05 (m,2H,CH₂NH) ; 5,30 (bs, 1H,NH) ; 5,45 (t, 1H,NCH(OCH₃)CH₂) | |
| 18 | 125 | | |
| 19 | 104 | | |
| 20 | 146 | | |
| 21 | 147 | | |
| 22 | 108 | | |
| 23 | 131 | | |
| 24 | 136 | | |
| 25 | 174 | | |
| 26 | 139 | | |
| 27 | 125 | | |
| 28 | 70 | | |
| 29 | 123 | | |
| 30 | 141 | | |
| 31 | 131 | | |
| 32 | 182 | | |
| 33 | 96 | | |
| 34 | 115 | | |
| 35 | 96 | | |
| 36 | | 1,15 (s,9H,ArC(CH₃)₃) ; 2,15 (s,3H,Ar(CH₃)) ; 2,20 (s,3H,Ar(CH₃)) | |
| 37 | | 2,17 (s,3H,ArCH₃) ; 2,20 (s,3H,ArCH₃) ; 3,13 (s,6H,N(CH₃)₂) | |
| 38 | | 1,10 (d,6H,CH(CH₃)₂) ; 1,30 (s,9H,(CH₃)₃) ; 2,15 (s,3H,ArCH₃) ; 2,20 (s,3H,ArCH₃) ; 4,00 (m,1H,CH(CH₃)₂) ; 4,45 (d,1H,CONH) ; 5,95 (s,1H,ArNH) | |
| 39 | | | 384 |
| 40 | 147 | | |
| 41 | 88 | | |
| 42 | 191 | | |
| 43 | 122 | | |
| 44 | | 1,30 (s,9H,(CH₃)₃) ; 2,05 (s,3H,ArCH₃) ; 2,25 (s,3H,ArCH₃) ; 3,40 (s,3H,NCH₃) ; 4,25 (s,2H,COCH₂N) | |
| 45 | | 1,30 (s,9H,(CH₃)₃) ; 1,95 (s,3H,ArCH₃) ; 2,15 (s,3H,ArCH₃) ; 4,20 (s,2H,COCH₂S) | |
| 46 | | 1,30 (s,9H,(CH₃)₃) ; 1,80 (t,3H,COCH(CH₃)S) ; 1,95 (s,3H,ArCH₃) ; 2,30 (s,3H,ArCH₃) ; 4,40 (m,1H,COCH(CH₃)S) | |
| 47 | 63 | | |
| 48 | 134 | | |
| 49 | 89 | | |
| 50 | 136 | | |
| 51 | 164 | | |
| 52 | 122 | | |
| 53 | | 1,25 (s,9H,C(CH₃)₃) ; 2,15 (s,3H,ArCH₃) ; 2,20 (s,3H,ArCH₃) | |
| 54 | | 1,20 (t,3H,CH₂CH₃) ; 1,25 (s,9H,C(CH₃)₃) ; 2,15 (s,3H,ArCH₃) ; 2,20 (s,3H,ArCH₃) ; 2,75 (s,3H,NCH₃) ; 3,90 (m,2H,NCH₂) ; 4,3 (m,4H,NCH₂N) | |
| 55 | | 1,15 (m,6H,2CH₂CH₃) ; 1,20 (s,9H,C(CH₃)₃) ; 2,15 (s,3H,AtCH₃) ; 2,20 (s,3H,ArCH₃) ; 2,95 (m,2H,CH₂CH₃) ; 3,9 (m,2H,NCH₂CH₃) ; 4,4 (m,4H,2NCH₂N) | |
| 56 | | 1,1 (t,3H,CH₂CH₃) ; 1,25 (s,9H,C(CH₃)₃) ; 2,10 (s,3H,ArCH₃) ; 2,20 (s,3H,ArCH₃) ; 3,8 (q,2H,NCH₂) | |
| 57 | | | 401 |
| 58 | | | 417 |
| 59 | | | 711 |
| 60 | | | 383 |
| 61 | | | 355 |
| 62 | | | 427 |
| 63 | | | 381 |
| 64 | | | 405 |
| 65 | | 3,44 (q,2H,NCH₂) ; 3,03 (s,3H,NCH₃) ; 2,19 (s,3H,ArCH₃) ; 2,12 (s,3H,ArCH₃) ; 1,22 (t,3H,NCH₂CH₃) | |
| 66 | | 3,88 (s,2H,NNH₂) ; 3,24 (s,3H,NCH₃) ; 2,19 (s,3H,ArCH₃) ; 2,12 (s,3H,ArCH₃) | |
| 67 | | 8,25 (s,2H,CONH₂) ; 2,22 (s,6H,ArCH₃) ; 2,07 (s,6H,ArCH₃) | |
| 68 | | 2,05 (s,3H,ArCH₃) ; 2,16 (s,3H,ArCH₃) | |
| 69 | | 2,79 (M,4H,NCH₂) ; 2,17 (s,3H,ArCH₃) ; 2,05 (s,3H,ArCH₃) | |
| 70 | | 2,10 (s,3H,ArCH₃) ; 2,04 (s,3H,ArCH₃) | |
| 71 | | 2,14 (s,3H,ArCH₃) ; 2,05 (s,3H,ArCH₃) | |
| 72 | | 2,07 (s,3H,ArCH₃) ; 2,03 (s,3H,ArCH₃) | |
| 73 | | 2,16 (s,3H,ArCH₃) ; 2,05 (s,3H,ArCH₃) | |
| 74 | | 3,60 (s,3H,OCH₃) ; 2,14 (s,3H,ArCH₃) ; 2,05 (s,3H,ArCH₃) | |
| 75 | | 2,14 (s,3H,ArCH₃) ; 2,05 (s,3H,ArCH₃) | |
| 76 | | 2,14 (s,3H,ArCH₃) ; 2,05 (s,3H,ArCH₃) ; 1,86 (s,3H,COCH₃) | |
| 77 | | 2,15 (s,3H,ArCH₃) ; 2,07 (s,3H,ArCH₃) | |
| 78 | | 2,17(s,3H,ArCH₃) ; 2,07 (s,3H,ArCH₃) | |
| 79 | | 3,04 (m,4H,NCH₂) ; 2,22 (s,3H,ArCH₃) ; 2,17 (s,3H,ArCH₃) | |
| 80 | | 2,22 (s,3H,ArCH₃) ; 2,15 (s,3H,ArCH₃) ; 1,21 (d,6H,NCH(CH₃)) | |
| 81 | | 2,13 (s,3H,ArCH₃) ; 2,07 (s,3H,ArCH₃) | |
| 82 | | 2,06 (s,3H,ArCH₃) ; 2,05 (s,3H,ArCH₃) | |
| 83 | | 2,16 (s,3H,ArCH₃) ; 2,07 (s,3H,ArCH₃) | |
| 84 | | 2,60 (s,3H,OCH₃) ; 2,13 (s,3H,ArCH₃) ; 2,07 (s,3H,ArCH₃) | |
| 85 | | 2,16 (s,3H,ArCH₃) ; 2,07 (s,3H,ArCH₃) | |
| 86 | | 2,13 (s,3H,ArCH₃) ; 2,06 (s,3H,ArCH₃) ; 1,86 (s,3H,COCH₃) | |
| 87 | | 2,64 (s,6H,NCH₃) ; 2,21 (s,3H,ArCH₃) ; 2,16 (s,3H,ArCH₃) | |
| 88 | | 3,24 (s,3H,NCH₃) ; 2,19 (s,3H,ArCH₃) ; 2,13 (s,3H,ArCH₃) | |
| 89 | | 3,11 (q,2H,NCH2CH₃) ; 2,14 (s,3H,ArCH₃) ; 2,04 (s,3H,ArCH₃) ; 1,07 (t,3H,NCH₂CH₃) | |
| 90 | | 3,75 (m,1H,NCH(CH₃)₂) ; 2,13 (s,3H,ArCH₃) ; 2,04 (s,3H,ArCH₃) ; 1,11 (d,6H,NCH(CH₃)₂) | |
| 91 | | 2,55 (m,1H,NHCH(CH₂)₂) ; 2,14 (s,3H,ArCH₃) ; 2,05 (s,3H,ArCH₃) ; 0,65 (m,2H,NCH(CH₂)₂) ; 0,42 (m,2H,NCH(CH₂)₂) | |
| 92 | | 3,42 (m,4H,NCH₂) ; 2,13 (s,3H,ArCH₃) ; 2,07 (s,3H,ArCH₃) | |
| 93 | | 3,40 (t,2H,OCH₂) ; 3,30 (s,3H,OCH₃) ; 3,27 (t,2H,NCH₂) ; 2,14 (s,3H,ArCH₃) ; 2,04 (s,3H,ArCH₃) | |
| 94 | | 2,22 (s,3H,ArCH₃) ; 2,08 (s,3H,ArCH₃) | |
| 95 | | 4,45 (d,2H,NHCH₂Ph) ; 2,16 (s,3H,ArCH₃) ; 2,05 (s,3H,ArCH₃) | |
| 96 | | 4,82 (q,1H,NCH) ; 2,15 (s,3H,ArCH₃) ; 2,02 (s,3H,ArCH₃) ; 1,40 (d,3H,NCH(CH₃) | |
| 97 | | 3,93 (t,4H,NCH₂) ; 2,15 (s,3H,ArCH₃) ; 2,0 (s,3H,ArCH₃) | |
| 98 | | 3,62 (m,4H,NCH₂) ; 3,42 (m,4H,CH₂O) ; 2,13 (s,3H,ArCH₃) ; 2,07 (s,3H,ArCH₃) | |
| 99 | | 2,29 (s,3H,ArCH₃) ; 2,10 (s,3H,ArCH₃) | |
| 100 | | 2,23 (s,3H,ArCH₃) ; 2,08 (s,3H,ArCH₃) | |
| 101 | | 3,89 (s,3H,OCH₃) ; 2,22 (s,3H,ArCH₃) ; 2,08 (s,3H,ArCH₃) | |
| 102 | | 2,22 (s,3H,ArCH₃) ; 2,09 (s,3H,ArCH₃) | |
| 103 | | 9,36 (s,1H,CCHN) ; 2,22 (s,3H,ArCH₃) ; 2,09 (s,3H,ArCH₃) | |
| 104 | | 3,95 (m,2H,CH₂CF₃) ; 2,14 (s,3H,ArCH₃) ; 2,04 (s,3H,ArCH₃) | |
| 105 | | 2,92 (S,3H,NCH₃) ; 2,09 (s,3H,ArCH₃) ; 2,04 (s,3H,ArCH₃) ; 1,34 (s,9H,NCCH₃) | |
| 106 | | 2,32 (s,3H,ArCH₃) ; 2,11 (s,3H,ArCH₃) | |
| 107 | | 3,67 (s,3H,OCH₃) ; 2,16 (s,3H,ArCH₃) ; 2,08 (s,3H,ArCH₃) | |
| 108 | | 4,12 (q,2H,OCH₂) ; 2,16 (s,3H,ArCH₃) ; 2,08 (s,3H,ArCH₃) ; 1,25 (t,3H,OCH2CH₃) | |
| 109 | | 4,87 (m,1H,OCH) ; 2,15 (s,3H,ArCH₃) ; 2,08 (s,3H,ArCH₃) ; 1,26 (t,6H,OCH(CH₃)₃) | |
| 110 | | 5,14 (s,2H,OCH₂Ph) ; 2,14 (s,3H,ArCH₃) ; 2,06 (s,3H,ArCH₃) | |
| 111 | | | 389 |
| 112 | | | 465 |
| 113 | | | 403 |
| 114 | | | 415 |
| 115 | | | 431 |
| 116 | | 3,40 (q,2H,ArNCH₂) ; 3,04 (q,2HCONCH₂) ; 2,46 (s,3H,NCH₃) ; 2,13 (s,3H,ArCH₃) ; 2,08 (s,3H,ArCH₃) ; 1,05 (t,3H,ArNCH₂CH₃) ; 0,77 (t,3H,CONCH₂CH₃) | 429 |
| 117 | | 4,56 (s,2H,ArCH₂N) ; 3,06 (q,2H,NCH₂) ; 2,49 (s,3H,NCH₃) ; 1,99 (s,3H,ArCH₃) ; 1,93 (s,3H,ArCH₃) ; 0,73 (t,3H,NCH₂CH₃) | |
| 118 | | 3,05 (q,2H,NCH2CH₃) ; 2,90 (s,3H,ArNCH₃) ; 2,46 (s,3H,EtNCH₃) ; 2,13 (s,3H,ArCH₃) ; 2,06 (s,3H,ArCH₃) ; 0,76 (t,3H,NCH₂CH₃) | |
| 119 | | 4,18 (s,2H,CH₂N) ; 3,02 (q,2H,NCH₂) ; 2,50 (s,3H,NCH₃) ; 2,19 (s,3H,ArCH₃) ; 2,11 (s,3H,ArCH₃) ; 1,00 (t,3H,NCH₂CH₃) | |
| 120 | | 3,94 (d,2H,CH₂CHCH₂) ; 3,04 (q,2H,NCH₂CH₃) ; 2,47 (s,3H,NCH₃) ; 2,10 (s,3H,ArCH3) ; 2,05 (s,3H,ArCH₃) ; 0,76 (t,3H,NCH₂CH₃) | |
| 121 | | 5,30 (q,1H,NCH) ; 4,03 (q,2H,NCH₂CH₃) ; 2,47 (s,3H,NCH₃) ; 2,01 (s,3H,ArCH₃) ; 1,82 (s,3H,ArCH₃) ; 1,40 (d,3H,NCH₂CH₃) ; 0,63 (t,3H,NCH₂CH₃) | |
| 122 | | | 373 |
| 123 | | 4,83 (m,3H,NCH₃S) ; 3,97 (m,2H,NCH₂) ; 3,18 (m,2H,SCH₂) ; 2,22 (s,3H,ArCH₃) ; 2,17 (s,3H,ArCH₃) ; 1,31 (s,9H,C(CH₃)₃) | |
| 124 | | 5,37 (m,1H,NCH) ; 3,00 (s,3H,NCH₃) ; 2,09 (s,3H,ArCH₃) ; 2,06 (s,3H,ArCH₃) ; 1,11 (d,6H,NCH(CH₃)₂) | |
| 125 | | | 386 |
| 126 | | 4,74 (d,2H,NCH₂Ph) ; 2,07 (s,3H,ArCH₃) ; 1,80 (s,3H,ArCH₃) ; 1,25 (s,9H,CCH₃) | |
| 127 | | | 384 |
| 128 | | | 388 |
| 129 | | | 436 |
| 130 | | | 413 |
| 131 | | | 444 |
| 132 | | | 446 |
| 133 | | | 432 |
| 134 | | | 466 |
| 135 | | | 430 |
| 136 | | | 416 |
| 137 | | | 459 |
| 138 | | | 434 |
| 139 | | | 482 |
| 140 | | | 427 |
| 141 | | | 429 |
| 142 | | | 415 |
| 143 | | | 449 |
| 144 | | | 413 |
| 145 | | | 399 |
| 146 | | | 442 |
| 147 | | | 401 |
| 148 | | | 417 |
| 149 | | | 401 |
| 150 | | | 465 |
| 151 | | | 464 |
| 152 | | | 401 |
| 153 | | | 432 |
| 154 | | | 415 |
| 155 | | | 386 |
| 156 | | | 404 |
| 157 | | | 387 |
| 158 | | | 404 |
| 159 | | | 329 |
| 160 | | | 376 |
| 161 | | | 421 |
| 162 | | | 467 |
| 163 | | | 450 |
| 164 | | | 370 |
| 165 | | | 416 |
| 166 | | | 452 |
| 167 | | 4,05 (t,4H,NCH₂) ; 2,07 (s,3H,ArCH₃) ; 1,80 (s,3H,ArCH₃) | |
| 168 | | 2,07 (s,3H,ArCH₃) ; 1,80 (s,3H,ArCH₃) | |
| 169 | | | 461 |
| 170 | | | 387 |
| 171 | | | 360 |

### Exemples Tests

Les composés ont été évalués pour leur activité contre une ou plusieurs des maladies fongiques suivantes :
*Phytophthora infestans* : mildiou de la tomate
*Erysiphe graminis f*. *sp*. *Tritici* : oïdium du blé
*Pyricularia oryzae* : pyriculariose du riz
*Leptosphaeria nodorum* : septoriose du blé, variété nodorum
*Mycosphaerella graminicola* : septoriose du blé, variété tritici
*Puccinia recondita* : rouille brune des céréales
*Pyrenophora teres* : helminthosporiose de l'orge

Des solutions ou dispersions aqueuses des composés à la concentration désirée, incluant un ou plusieurs agents mouillants, sont appliquées par pulvérisation ou par trempage de la base de la tige des plantes test, selon le cas. Après un temps donné, les plants ou parties des plants sont inoculés avec les pathogènes tests appropriés, et conservés dans des conditions environnementales contrôlées convenables pour maintenir la croissance de la plante et le développement de la maladie. Après un temps approprié, le degré d'infection de la partie infectée de la plante est estimé visuellement. À une concentration de 500 ppm (poids/volume) ou moins, les composés suivants présentent contre les maladies fongiques spécifiées un contrôle d'au moins 65% d'efficacité par rapport à un témoin non traité.

### Phytophthora infestans

44, 45

### Erysiphe graminis f. sp. Tritici

1, 17, 18, 19, 20, 21, 22, 23, 24, 27, 28, 33, 42, 43, 45, 52, 53, 54, 55, 83, 84, 124, 170

### Pyricularia oryzae

35

### Leptosphaeria nodorum

18, 27, 45, 124, 169, 170, 171

### Puccinia recondita

124, 127, 131, 140, 149, 157, 167, 169, 170

### Pyrenophora teres

77, 93, 95, 113, 118, 119, 124, 128, 132, 140, 144, 145, 149, 150, 157, 164, 165

## Revendications

1. Composés de formule générale (I) ainsi que leurs sels : 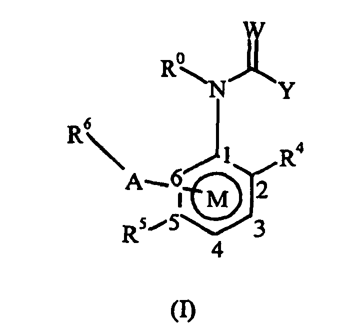 dans laquelle
• W représente O ou S ;
• Y représente un radical -NR¹R², -OR³ ou -SR³ ;
• R⁰ représente un radical alkyle ou l'atome d'hydrogène ;
• R¹ et R², qui peuvent être identiques ou différents, représentent un radical alkyle ou acyle chacun d'entre eux pouvant être substitué par un alkoxy ; ou l'atome d'hydrogène ; ou -NR^{a}R^{b} , -OR^{a} dans lesquels R^{a} et R^{b}, qui peuvent être identiques ou différents, représentent un radical alkyl, acyle ou carbocyclyle, chacun d'entre eux pouvant être substitué ;
• R³ représente un radical alkyle ou acyle chacun d'entre eux pouvant être substitué par un alkoxy ; ou l'atome d'hydrogène ;
• R¹ et R² ou R¹ et R⁰ ou R³ et R⁰ ou R^{a} et R^{b}, pris ensemble avec les atomes les connectant, peuvent former un cycle, éventuellement substitué, l'ensemble formant alors un groupement carbocyclyle ou hétérocyclyle ;
• R⁴ représente un radical alkyle pouvant être substitué par un atome d'halogène ;
• R⁵ représente un radical alkyle ou acyle chacun d'entre eux pouvant être substitué; ou l'atome d'hydrogène ;
• R⁶ représente un radical phényle ou un radical hétérocyclyle aromatique ;
• A représente -O- ou -S-.

2. Composés selon la revendication 1 dans lesquels :
• R⁰ représente un radical alkyle ou représente l'atome d'hydrogène ;
• R¹, R² et R³ sont tels que définis précédemment ;
• R⁴ représente un radical alkyle pouvant être substitué par un atome d'halogène ;
• R⁵ représente un radical alkyle pouvant être substitué par un atome d'halogène ;
• A représente les radicaux divalents -O- ou -S- ; et
• R⁶ représente un radical phényle ou hétérocyclyle aromatique.

3. Composés selon l'une des revendications précédentes possédant les caractéristiques suivantes prises isolément ou en combinaison :
• R⁴ représente un radical alkyle en C₁-C₁₀ ;
• R⁵ représente un radical alkyle en C₁-C₁₀ ;
• A représente le radical divalent -O-, et occupe la position 4 sur le noyau benzénique M ;
• R⁶ représente un radical phényle.

4. Utilisation des composés selon l'une quelconque des revendications précédentes comme fongicides.

5. Composition fongicide comprenant au moins un composé selon l'une quelconque des revendications 1 à 3 ou un de ses sels en mélange avec un diluant ou un support acceptable en agriculture.

6. Méthode pour combattre les champignons phytopathogènes en un lieu qui en est infesté ou susceptible de l'être, qui comprend l'application en ce lieu d'un composé selon l'une quelconque des revendications 1 à 3 ou d'un de ses sels.

## Claims

1. Compounds of general formula (I) and the salts thereof: 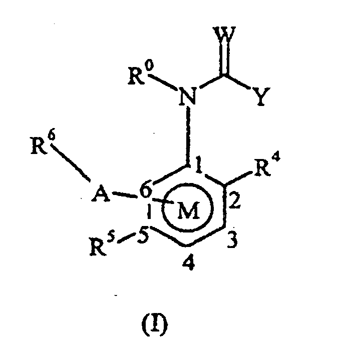 in which
• W represents O or S;
• Y represents a radical -NR¹R², -OR³ or -SR³;
• R° represents an alkyl radical or a hydrogen atom;
• R¹ and R², which may be identical or different, represent an alkyl or acyl radical, each of which may be substituted with an alkoxy; or a hydrogen atom; or -NR^{a}R^{b}, -OR^{a} in which R^{a} and R^{b}, which may be identical or different, represent an alkyl, acyl or carbocyclyl radical, each of which may be substituted;
• R³ represents an alkyl or acyl radical, each of which may be substituted with an alkoxy; or a hydrogen atom;
• R¹ and R² or R¹ and R⁰ or R³ and R⁰ or R^{a} and R^{b}, taken together with the atoms connecting them, may form an optionally substituted ring, the assembly thus forming a carbocyclyl or heterocyclyl group;
• R⁴ represents an alkyl radical which may be substituted with a halogen atom;
• R⁵ represents an alkyl or acyl radical, each of which may be substituted; or a hydrogen atom;
• R⁶ represents a phenyl radical or an aromatic heterocyclyl radical;
• A represents -O- or -S-.

2. Compounds according to Claim 1, in which:
• R⁰ represents an alkyl radical or represents a hydrogen atom;
• R¹, R² and R³ are as defined above;
• R⁴ represents an alkyl radical which may be substituted with a halogen atom;
• R⁵ represents an alkyl radical which may be substituted with a halogen atom;
• A represents the divalent radicals -O- or -S-; and
• R⁶ represents a phenyl or aromatic heterocyclyl radical.

3. Compounds according to either of the preceding claims, which have the following characteristics taken separately or in combination:
• R⁴ represents a C₁-C₁₀ alkyl radical;
• R⁵ represents a C₁-C₁₀ alkyl radical;
• A represents the divalent radical -O-, and occupies position 4 on the benzene nucleus M;
• R⁶ represents a phenyl radical.

4. Use of the compounds according to any one of the preceding claims, as fungicides.

5. Fungicidal composition comprising at least one compound according to any one of Claims 1 to 3, or a salt thereof, mixed with an agriculturally acceptable diluent or support.

6. Method for combating phytopathogenic fungi at a site which is infested or liable to be infested with them, which comprises the application at this site of a compound according to any one of Claims 1 to 3, or a salt thereof.

## Patentansprüche

1. Verbindungen der allgemeinen Formel (I) sowie ihre Salze: 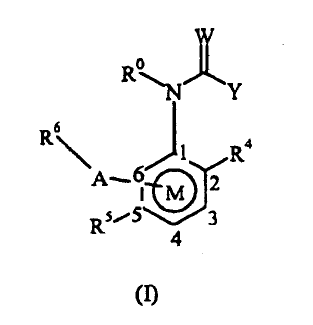 worin
• W für O oder S steht;
• Y für einen Rest -NR¹R², -OR³ oder -SR³ steht;
• R⁰ für einen Alkylrest oder ein Wasserstoffatom steht;
• R¹ und R², die gleich oder verschieden sein können, für einen Alkyl- oder Acylrest, von denen jeder mit einem Alkoxy substituiert sein kann; oder ein Wasserstoffatom; oder NR^{a}R^{b}, OR^{a} stehen, worin R^{a} und R^{b}, die gleich oder verschieden sein können, für einen Alkyl-, Acyl- oder Carbocyclylrest stehen, von denen jeder substituiert sein kann;
• R³ für einen Alkyl- oder Acylrest, von denen jeder mit einem Alkoxy substituiert sein kann; oder ein Wasserstoffatom steht;
• R¹ und R² oder R¹ und R⁰ oder R³ und R⁰ oder R^{a} und R^{b} zusammen mit den Atomen, die sie verbinden, einen gegebenenfalls substituierten Ring bilden können, wobei die Gesamtheit so eine Carbocyclyl- oder Heterocyclylgruppe bildet;
• R⁴ für einen Alkylrest steht, der mit einem Halogenatom substituiert sein kann;
• R⁵ für einen Alkyl- oder Acylrest, von denen jeder substituiert sein kann; oder ein Wasserstoffatom steht;
• R⁶ für einen Phenylrest oder einen aromatischen Heterocyclylrest steht;
• A für -O- oder -S- steht.

2. Verbindungen nach Anspruch 1, worin:
• R° für einen Alkylrest oder ein Wasserstoffatom steht;
• R¹, R² und R³ wie vorstehend definiert sind;
• R⁴ für einen Alkylrest steht, der mit einem Halogenatom substituiert sein kann;
• R⁵ für einen Alkylrest steht, der mit einem Halogenatom substituiert sein kann;
• A für die zweiwertigen Reste -0- oder -S- steht; und
• R⁶ für einen Phenylrest oder einen aromatischen Heterocyclylrest steht.

3. Verbindungen nach einem der vorstehenden Ansprüche, die die folgenden Eigenschaften allein oder in Kombination besitzen:
• R⁴ steht für einen C₁-C₁₀-Alkylrest;
• R⁵ steht für einen C₁-C₁₀-Alkylrest;
• A steht für den zweiwertigen Rest -O- und besetzt die Position 4 am Benzolkern M;
• R⁶ steht für einen Phenylrest.

4. Verwendung der Verbindungen nach einem der vorstehenden Ansprüche als Fungizide.

5. Fungizide Zusammensetzung, die mindestens eine Verbindung nach einem der Ansprüche 1 bis 3 oder eines ihrer Salze im Gemisch mit einem landwirtschaftlich annehmbaren Verdünnungsmittel oder Träger umfasst.

6. Verfahren zur Bekämpfung phytopathogener Pilze an einer Stelle, die davon befallen ist oder empfänglich dafür ist, davon befallen zu werden, umfassend das Aufbringen einer Verbindung nach einem der Ansprüche 1 bis 3 oder eines ihrer Salze auf diese Stelle.
